(19) Europäisches Patentamt
European Patent Office
Office européen des brevets

(11) EP 1 600 458 A1

(12) EUROPEAN PATENT APPLICATION
published in accordance with Art. 158(3) EPC

(43) Date of publication:
**30.11.2005 Bulletin 2005/48**

(21) Application number: **04716792.9**

(22) Date of filing: **03.03.2004**

(51) Int Cl.[7]: **C07K 14/47**, C12N 15/09, C12N 1/15, C12N 1/19, C12N 1/21, C12N 5/06, C12Q 1/02, C12Q 1/68, A61K 38/17, A61P 13/00, A61P 13/12, A61P 43/00, G01N 33/50, G01N 33/15

(86) International application number:
**PCT/JP2004/002648**

(87) International publication number:
**WO 2004/078784 (16.09.2004 Gazette 2004/38)**

(84) Designated Contracting States:
**AT BE BG CH CY CZ DE DK EE ES FI FR GB GR HU IE IT LI LU MC NL PL PT RO SE SI SK TR**
Designated Extension States:
**AL LT LV MK**

(30) Priority: **04.03.2003 JP 2003056522**
**26.12.2003 JP 2003433586**

(71) Applicant: **Astellas Pharma Inc.**
**Tokyo 103-8411 (JP)**

(72) Inventors:
- **OGINO, Makoto,**
**Yamanouchi Pharmaceutical Co., Ltd.**
**Tsukuba-shi, Ibaraki 3058585 (JP)**
- **HIROSE, Tomohiro,**
**Yamanouchi Pharmac. Co. Ltd.**
**Tsukuba-shi, Ibaraki 3058585 (JP)**
- **HAYASHI, Kazumi,**
**Yamanouchi Pharmaceutical Co. Ltd**
**Tsukuba-shi, Ibaraki 3058585 (JP)**
- **KOIZUMI, Tomonobu,**
**Yamanouchi Pharma. Co. Ltd.**
**Tsukuba-shi, Ibaraki 3058585 (JP)**
- **YOKOYAMA, Toshihide,**
**Yamanouchi Pharma. Co. Ltd.**
**Tsukuba-shi, Ibaraki 3058585 (JP)**

(74) Representative: **Bates, Philip Ian**
**Reddie & Grose**
**16 Theobalds Road**
**London WC1X 8PL (GB)**

(54) **NOVEL GENE RELATING TO FIBROTIC CONDITIONS**

(57) This discloses a novel human or rat polypeptide useful in improving and/or preventing organ fibrosis and a polynucleotide encoding the aforementioned polypeptide, an expression vector containing the aforementioned polynucleotide, and a cell transfected with the aforementioned expression vector. The aforementioned polynucleotide shows a remarkable decrease in the expression dose in the kidney of a chronic renal insufficiency model rat and in the bladder of a rat suffering from the induction of fibrotic conditions in the bladder, compared with a normal individual. Also, the production of extracellular matrix is suppressed by overexpression of the aforementioned polypeptide. Also disclosed are the promoter region of the aforementioned polypeptide, a method for screening a remedy for fibrotic conditions, and a process for producing a medicinal composition for improving fibrotic conditions which contains a substance obtained by the aforementioned screening method as the active ingredient.

EP 1 600 458 A1

SEQUENCE LISTING

<110> Yamanouchi Pharmaceutical Co.,Ltd

<120> Novel fibrosis related gene

<130> Y0406-PCT

<150> JP2003-056522
<151> 2003-03-04

<150> JP2003-433586
<151> 2003-12-26

<160> 25

<170> PatentIn version 3.1

<210> 1
<211> 1908
<212> DNA
<213> Homo sapiens

<220>
<221> CDS
<222> (1)..(1905)
<223> Inventor; Ogino, Makoto; Hirose, Tomohiro; Yokoyama, Toshihide; Koizumi, Tomonobu, Hayashi kazumi

<400> 1
```
atg gca tgg ccc aaa ctg ccc gca cct tgg ctg ctg ctc tgc acc tgg        48
Met Ala Trp Pro Lys Leu Pro Ala Pro Trp Leu Leu Leu Cys Thr Trp
1               5                   10                  15

ctc cca gca ggg tgc ctg tcc ttg ctt gtg acg gtc cag cac aca gaa        96
```

Leu Pro Ala Gly Cys Leu Ser Leu Leu Val Thr Val Gln His Thr Glu
20 25 30

cgc tat gtc acc ctg ttt gcc tct atc atc ctc aaa tgt gac tac acc 144
Arg Tyr Val Thr Leu Phe Ala Ser Ile Ile Leu Lys Cys Asp Tyr Thr
35 40 45

acc tct gcc cag ctc cag gac gtg gtg gtg aca tgg cgc ttc aag tcc 192
Thr Ser Ala Gln Leu Gln Asp Val Val Val Thr Trp Arg Phe Lys Ser
50 55 60

ttc tgc aag gac cct atc ttt gac tac tac tca gcg tca tac cag gca 240
Phe Cys Lys Asp Pro Ile Phe Asp Tyr Tyr Ser Ala Ser Tyr Gln Ala
65 70 75 80

gct tta tcc ctg ggc cag gac cca tcc aat gac tgc aac gac aac cag 288
Ala Leu Ser Leu Gly Gln Asp Pro Ser Asn Asp Cys Asn Asp Asn Gln
85 90 95

cgg gaa gtt cgc ata gtg gcc cag cgg cgg ggg cag aat gag ccc gtg 336
Arg Glu Val Arg Ile Val Ala Gln Arg Arg Gly Gln Asn Glu Pro Val
100 105 110

ctg ggg gta gat tac cgg cag cgc aag atc acc atc cag aac cga gca 384
Leu Gly Val Asp Tyr Arg Gln Arg Lys Ile Thr Ile Gln Asn Arg Ala
115 120 125

gat ctc gtg ata aat gaa gtg atg tgg tgg gac cat gga gtg tat tac 432
Asp Leu Val Ile Asn Glu Val Met Trp Trp Asp His Gly Val Tyr Tyr
130 135 140

tgc acc att gag gct cca ggg gac aca tca gga gac ccc gat aag gaa 480
Cys Thr Ile Glu Ala Pro Gly Asp Thr Ser Gly Asp Pro Asp Lys Glu
145 150 155 160

gta aag ctc atc gtc cta cac tgg ctg aca gtg atc ttc atc atc ctg 528

Val Lys Leu Ile Val Leu His Trp Leu Thr Val Ile Phe Ile Ile Leu
165 170 175

gga gcc ctc ctc ctc ctg ctg ctg att gga gtg tgc tgg tgc cag tgc 576
Gly Ala Leu Leu Leu Leu Leu Leu Ile Gly Val Cys Trp Cys Gln Cys
180 185 190

tgt cct cag tat tgc tgc tgc tat atc cgc tgt ccc tgc tgt cct gcc 624
Cys Pro Gln Tyr Cys Cys Cys Tyr Ile Arg Cys Pro Cys Cys Pro Ala
195 200 205

cac tgc tgc tgt cct gaa gaa gcc ctg gcc cgc cac cgc tac atg aag 672
His Cys Cys Cys Pro Glu Glu Ala Leu Ala Arg His Arg Tyr Met Lys
210 215 220

cag gcc cag gcc cta ggt cct cag atg atg gga aaa ccc ctg tac tgg 720
Gln Ala Gln Ala Leu Gly Pro Gln Met Met Gly Lys Pro Leu Tyr Trp
225 230 235 240

ggg gcg gac agg agc tcc cag gtt tca tct tat cca atg cac ccg ctg 768
Gly Ala Asp Arg Ser Ser Gln Val Ser Ser Tyr Pro Met His Pro Leu
245 250 255

ctg cag cga gat ttg tcc ctg ccg tcc agc ctc ccg cag atg cca atg 816
Leu Gln Arg Asp Leu Ser Leu Pro Ser Ser Leu Pro Gln Met Pro Met
260 265 270

acc cag acc acc aat cag cct ccc atc gcc aat ggt gtc ctg gag tat 864
Thr Gln Thr Thr Asn Gln Pro Pro Ile Ala Asn Gly Val Leu Glu Tyr
275 280 285

ttg gag aaa gaa ctg cgg aac ctc aac ctg gcc cag cct ctg ccc cct 912
Leu Glu Lys Glu Leu Arg Asn Leu Asn Leu Ala Gln Pro Leu Pro Pro
290 295 300

gac ctc aaa ggc aga ttt ggc cat ccc tgc agc atg ctg tcc tcc ctg 960

Asp Leu Lys Gly Arg Phe Gly His Pro Cys Ser Met Leu Ser Ser Leu
305 310 315 320

ggc tct gag gtc gtg gaa cgc aga atc atc cac ctg ccc cca ctg atc 1008
Gly Ser Glu Val Val Glu Arg Arg Ile Ile His Leu Pro Pro Leu Ile
325 330 335

aga gac ctg tca tcc tca agg agg acc agt gac tcc ctg cac cag cag 1056
Arg Asp Leu Ser Ser Ser Arg Arg Thr Ser Asp Ser Leu His Gln Gln
340 345 350

tgg ctc acc cca att ccc tcc agg ccc tgg gat ctg agg gag ggg aga 1104
Trp Leu Thr Pro Ile Pro Ser Arg Pro Trp Asp Leu Arg Glu Gly Arg
355 360 365

agc cac cac cat tac cct gat ttc cac cag gag ctc cag gac cgg ggg 1152
Ser His His His Tyr Pro Asp Phe His Gln Glu Leu Gln Asp Arg Gly
370 375 380

cca aag tct tgg gca ttg gaa aga agg gag ttg gac cca tcg tgg agt 1200
Pro Lys Ser Trp Ala Leu Glu Arg Arg Glu Leu Asp Pro Ser Trp Ser
385 390 395 400

gga agg cac cgt agc tct agg ctg aat ggg tca ccc ata cac tgg tca 1248
Gly Arg His Arg Ser Ser Arg Leu Asn Gly Ser Pro Ile His Trp Ser
405 410 415

gac agg gac agc cta agc gat gtc ccc tca tcc agt gag gca cgc tgg 1296
Asp Arg Asp Ser Leu Ser Asp Val Pro Ser Ser Ser Glu Ala Arg Trp
420 425 430

cgg ccg agc cac cct cct ttc agg agc cgc tgt cag gag agg ccc cgc 1344
Arg Pro Ser His Pro Pro Phe Arg Ser Arg Cys Gln Glu Arg Pro Arg
435 440 445

agg ccc agc ccc cgg gag agc act cag agg cac ggg aga cga cgc agg 1392

Arg Pro Ser Pro Arg Glu Ser Thr Gln Arg His Gly Arg Arg Arg Arg
450 455 460

cac cgc agc tac tct cct ccc ttg ccc tcc ggc ctc agt tcc tgg agc 1440
His Arg Ser Tyr Ser Pro Pro Leu Pro Ser Gly Leu Ser Ser Trp Ser
465 470 475 480

tct gaa gag gac aag gag agg cag ccc cag agc tgg cgg gcc cac cgc 1488
Ser Glu Glu Asp Lys Glu Arg Gln Pro Gln Ser Trp Arg Ala His Arg
485 490 495

cgc ggc tcg cac tcc cca cac tgg ccc gag gag aag ccg cct agc tac 1536
Arg Gly Ser His Ser Pro His Trp Pro Glu Glu Lys Pro Pro Ser Tyr
500 505 510

cgc tca ctg gat atc act cca ggc aag aat agc agg aaa aaa ggg agt 1584
Arg Ser Leu Asp Ile Thr Pro Gly Lys Asn Ser Arg Lys Lys Gly Ser
515 520 525

gtg gag agg cgc tcg gtg agc ctg ggg cat cct gct gag ggt tgg gca 1632
Val Glu Arg Arg Ser Val Ser Leu Gly His Pro Ala Glu Gly Trp Ala
530 535 540

tgg gca gag agg agc ctc cag cca ggc atg acc aca gcc aac aca ggc 1680
Trp Ala Glu Arg Ser Leu Gln Pro Gly Met Thr Thr Ala Asn Thr Gly
545 550 555 560

tgc ctc tca ttc cac cac aga ggg tgc ctc ctc cct gtt ttg ccc aaa 1728
Cys Leu Ser Phe His His Arg Gly Cys Leu Leu Pro Val Leu Pro Lys
565 570 575

tta cac tgt ggg cta ggt gga cta cct ctt gtc aga gct aaa gaa atc 1776
Leu His Cys Gly Leu Gly Gly Leu Pro Leu Val Arg Ala Lys Glu Ile
580 585 590

aag cga gtg cag agg gca ggg gag agt tcg ctg cct gtg aag ggc ctt 1824

Lys Arg Val Gln Arg Ala Gly Glu Ser Ser Leu Pro Val Lys Gly Leu
595 600 605

ctc acc gtc gct tcg gct gtc atc gca gtc ctg tgg ggt agg cca agc 1872
Leu Thr Val Ala Ser Ala Val Ile Ala Val Leu Trp Gly Arg Pro Ser
610 615 620

gag gtc aca gga gaa aat gag gct cag cat gat taa 1908
Glu Val Thr Gly Glu Asn Glu Ala Gln His Asp
625 630 635

<210> 2
<211> 635
<212> PRT
<213> Homo sapiens

<400> 2

Met Ala Trp Pro Lys Leu Pro Ala Pro Trp Leu Leu Leu Cys Thr Trp
1 5 10 15

Leu Pro Ala Gly Cys Leu Ser Leu Leu Val Thr Val Gln His Thr Glu
20 25 30

Arg Tyr Val Thr Leu Phe Ala Ser Ile Ile Leu Lys Cys Asp Tyr Thr
35 40 45

Thr Ser Ala Gln Leu Gln Asp Val Val Val Thr Trp Arg Phe Lys Ser
50 55 60

Phe Cys Lys Asp Pro Ile Phe Asp Tyr Tyr Ser Ala Ser Tyr Gln Ala

65 70 75 80

Ala Leu Ser Leu Gly Gln Asp Pro Ser Asn Asp Cys Asn Asp Asn Gln
85 90 95

Arg Glu Val Arg Ile Val Ala Gln Arg Arg Gly Gln Asn Glu Pro Val
100 105 110

Leu Gly Val Asp Tyr Arg Gln Arg Lys Ile Thr Ile Gln Asn Arg Ala
115 120 125

Asp Leu Val Ile Asn Glu Val Met Trp Trp Asp His Gly Val Tyr Tyr
130 135 140

Cys Thr Ile Glu Ala Pro Gly Asp Thr Ser Gly Asp Pro Asp Lys Glu
145 150 155 160

Val Lys Leu Ile Val Leu His Trp Leu Thr Val Ile Phe Ile Ile Leu
165 170 175

Gly Ala Leu Leu Leu Leu Leu Leu Ile Gly Val Cys Trp Cys Gln Cys
180 185 190

Cys Pro Gln Tyr Cys Cys Cys Tyr Ile Arg Cys Pro Cys Cys Pro Ala
195 200 205

His Cys Cys Cys Pro Glu Glu Ala Leu Ala Arg His Arg Tyr Met Lys

210 215 220

Gln Ala Gln Ala Leu Gly Pro Gln Met Met Gly Lys Pro Leu Tyr Trp
225 230 235 240

Gly Ala Asp Arg Ser Ser Gln Val Ser Ser Tyr Pro Met His Pro Leu
245 250 255

Leu Gln Arg Asp Leu Ser Leu Pro Ser Ser Leu Pro Gln Met Pro Met
260 265 270

Thr Gln Thr Thr Asn Gln Pro Pro Ile Ala Asn Gly Val Leu Glu Tyr
275 280 285

Leu Glu Lys Glu Leu Arg Asn Leu Asn Leu Ala Gln Pro Leu Pro Pro
290 295 300

Asp Leu Lys Gly Arg Phe Gly His Pro Cys Ser Met Leu Ser Ser Leu
305 310 315 320

Gly Ser Glu Val Val Glu Arg Arg Ile Ile His Leu Pro Pro Leu Ile
325 330 335

Arg Asp Leu Ser Ser Ser Arg Arg Thr Ser Asp Ser Leu His Gln Gln
340 345 350

Trp Leu Thr Pro Ile Pro Ser Arg Pro Trp Asp Leu Arg Glu Gly Arg

355 360 365

Ser His His His Tyr Pro Asp Phe His Gln Glu Leu Gln Asp Arg Gly
370 375 380

Pro Lys Ser Trp Ala Leu Glu Arg Arg Glu Leu Asp Pro Ser Trp Ser
385 390 395 400

Gly Arg His Arg Ser Ser Arg Leu Asn Gly Ser Pro Ile His Trp Ser
405 410 415

Asp Arg Asp Ser Leu Ser Asp Val Pro Ser Ser Ser Glu Ala Arg Trp
420 425 430

Arg Pro Ser His Pro Pro Phe Arg Ser Arg Cys Gln Glu Arg Pro Arg
435 440 445

Arg Pro Ser Pro Arg Glu Ser Thr Gln Arg His Gly Arg Arg Arg Arg
450 455 460

His Arg Ser Tyr Ser Pro Pro Leu Pro Ser Gly Leu Ser Ser Trp Ser
465 470 475 480

Ser Glu Glu Asp Lys Glu Arg Gln Pro Gln Ser Trp Arg Ala His Arg
485 490 495

Arg Gly Ser His Ser Pro His Trp Pro Glu Glu Lys Pro Pro Ser Tyr

500 505 510

Arg Ser Leu Asp Ile Thr Pro Gly Lys Asn Ser Arg Lys Lys Gly Ser
515 520 525

Val Glu Arg Arg Ser Val Ser Leu Gly His Pro Ala Glu Gly Trp Ala
530 535 540

Trp Ala Glu Arg Ser Leu Gln Pro Gly Met Thr Thr Ala Asn Thr Gly
545 550 555 560

Cys Leu Ser Phe His His Arg Gly Cys Leu Leu Pro Val Leu Pro Lys
565 570 575

Leu His Cys Gly Leu Gly Gly Leu Pro Leu Val Arg Ala Lys Glu Ile
580 585 590

Lys Arg Val Gln Arg Ala Gly Glu Ser Ser Leu Pro Val Lys Gly Leu
595 600 605

Leu Thr Val Ala Ser Ala Val Ile Ala Val Leu Trp Gly Arg Pro Ser
610 615 620

Glu Val Thr Gly Glu Asn Glu Ala Gln His Asp
625 630 635

<210> 3

<211> 1623
<212> DNA
<213> Rattus sp.

<220>
<221> CDS
<222> (1)..(1620)
<223>

<400> 3

```
atg ggg gcc agc atg ggc tgc ggg ctg cta gtt gct ggc ctg ctc ctt       48
Met Gly Ala Ser Met Gly Cys Gly Leu Leu Val Ala Gly Leu Leu Leu
1               5                   10                  15

ttc acc tgg ctc cca gca ggg tgt ctg tcc ttg ctg gtc aca gtt cag       96
Phe Thr Trp Leu Pro Ala Gly Cys Leu Ser Leu Leu Val Thr Val Gln
            20                  25                  30

cac acc gaa cgc tat gtc act ctg ttt gcc tct gtc acc ctc aaa tgt      144
His Thr Glu Arg Tyr Val Thr Leu Phe Ala Ser Val Thr Leu Lys Cys
        35                  40                  45

gac tac acc acc tct gct cag ctc cag gat gtg gtt gtg aca tgg cgc      192
Asp Tyr Thr Thr Ser Ala Gln Leu Gln Asp Val Val Val Thr Trp Arg
    50                  55                  60

ttc aag tcc ttc tgc aag gac ccc atc ttt gac tac ttc tct gcc tca      240
Phe Lys Ser Phe Cys Lys Asp Pro Ile Phe Asp Tyr Phe Ser Ala Ser
65                  70                  75                  80

tac cag gcc gct ttg tcc ctg ggc cag gac ccc tcc aat gac tgt agt      288
Tyr Gln Ala Ala Leu Ser Leu Gly Gln Asp Pro Ser Asn Asp Cys Ser
                85                  90                  95

gac aat cag agg gaa gtt cgc atc gtg gcc cag cgg cgc ggg cag agt      336
Asp Asn Gln Arg Glu Val Arg Ile Val Ala Gln Arg Arg Gly Gln Ser
```

100 105 110

gag cca gtg ctg ggg gtg gat tac cgg cag cgc aag atc acc atc cag 384
Glu Pro Val Leu Gly Val Asp Tyr Arg Gln Arg Lys Ile Thr Ile Gln
115 120 125

aac cga gca gat ctt gtg att aat gaa gtg atg tgg tgg gat cat ggg 432
Asn Arg Ala Asp Leu Val Ile Asn Glu Val Met Trp Trp Asp His Gly
130 135 140

gta tac tat tgc acc atc gag gct cca gga gac aca tcg gga gac cca 480
Val Tyr Tyr Cys Thr Ile Glu Ala Pro Gly Asp Thr Ser Gly Asp Pro
145 150 155 160

gat aag gag gtg aag ctc atc gtg ctg cat tgg ctg aca gtg atc ttc 528
Asp Lys Glu Val Lys Leu Ile Val Leu His Trp Leu Thr Val Ile Phe
165 170 175

atc atc ctt gga gcc ctc ctt ctc ctg ctg ctg att ggt gtg tgc tgg 576
Ile Ile Leu Gly Ala Leu Leu Leu Leu Leu Leu Ile Gly Val Cys Trp
180 185 190

tgc cag tgt tgt ccg cag tat tgc tgc tgc tac atc cgc tgc ccc tgc 624
Cys Gln Cys Cys Pro Gln Tyr Cys Cys Cys Tyr Ile Arg Cys Pro Cys
195 200 205

tgt ccc acc tgc tgt tgc tgt ccc gag gaa gcc ctg gcc cgc cac cgc 672
Cys Pro Thr Cys Cys Cys Cys Pro Glu Glu Ala Leu Ala Arg His Arg
210 215 220

tac atg aag cag gta cag gcc cta ggt cct cag atg atg gaa aaa ccc 720
Tyr Met Lys Gln Val Gln Ala Leu Gly Pro Gln Met Met Glu Lys Pro
225 230 235 240

ctg tac tgg ggg gcg gac agg agc tcc caa gtt tca tct tat gca atg 768
Leu Tyr Trp Gly Ala Asp Arg Ser Ser Gln Val Ser Ser Tyr Ala Met

245 250 255

aac ccg ctg ctg cag cga gat ctg tcc tta cgg tcc agc ctt cca cag 816
Asn Pro Leu Leu Gln Arg Asp Leu Ser Leu Arg Ser Ser Leu Pro Gln
260 265 270

atg cca atg acc cag atg gcc gct cac cct ccc gtg gcc aat ggc gtc 864
Met Pro Met Thr Gln Met Ala Ala His Pro Pro Val Ala Asn Gly Val
275 280 285

ttg gaa tat ttg gag aaa gaa ttg cgg aac ctc aac cca gcc cag cct 912
Leu Glu Tyr Leu Glu Lys Glu Leu Arg Asn Leu Asn Pro Ala Gln Pro
290 295 300

ctg cct ccg gat ctc aga acc aag tcc ggt cac cct tgc agc atg ctc 960
Leu Pro Pro Asp Leu Arg Thr Lys Ser Gly His Pro Cys Ser Met Leu
305 310 315 320

tct tcc ctg ggc tcc gcc gag gtt gtg gaa cgc aga gtc atc cac ctg 1008
Ser Ser Leu Gly Ser Ala Glu Val Val Glu Arg Arg Val Ile His Leu
325 330 335

ccc cca ctg atc aga gac cca cag ccc tcc agg acc agc aac tcc tca 1056
Pro Pro Leu Ile Arg Asp Pro Gln Pro Ser Arg Thr Ser Asn Ser Ser
340 345 350

cac cag cag cgg ctc aat cct gtt cct tcc aga ccc tgg ggt ccg agt 1104
His Gln Gln Arg Leu Asn Pro Val Pro Ser Arg Pro Trp Gly Pro Ser
355 360 365

gag ggc cgg agg cag cgc aat cac tcg gat ttc ctc cga gaa ctc cag 1152
Glu Gly Arg Arg Gln Arg Asn His Ser Asp Phe Leu Arg Glu Leu Gln
370 375 380

gat cgg ggg atg aga ccc tgg gcc ccc ggg aga ggg gag ctg gac ccc 1200
Asp Arg Gly Met Arg Pro Trp Ala Pro Gly Arg Gly Glu Leu Asp Pro

385 390 395 400

cat tgg agt ggg aga cac cac cgc tct agg cct agc gag tcc tcc atg 1248
His Trp Ser Gly Arg His His Arg Ser Arg Pro Ser Glu Ser Ser Met
405 410 415

ccc tgg tca gac tgg gac agc ctg agc gaa tgt ccc tcg tcc agt gag 1296
Pro Trp Ser Asp Trp Asp Ser Leu Ser Glu Cys Pro Ser Ser Ser Glu
420 425 430

gct cct tgg ccc tcc aga cga cca gag ccc cgg gaa gga tcc cag aga 1344
Ala Pro Trp Pro Ser Arg Arg Pro Glu Pro Arg Glu Gly Ser Gln Arg
435 440 445

cat ggg aga cgc agg cat cgc agc tac tca cct ccc cta ccc tcg ggc 1392
His Gly Arg Arg Arg His Arg Ser Tyr Ser Pro Pro Leu Pro Ser Gly
450 455 460

ccc agt tct tgg agc tct gaa gag gag aaa gag tcg ctg cct agg aac 1440
Pro Ser Ser Trp Ser Ser Glu Glu Glu Lys Glu Ser Leu Pro Arg Asn
465 470 475 480

tgg ggt gcc cag cga cgt cac cat cgc cgc agc cgc cgc cgc tca cag 1488
Trp Gly Ala Gln Arg Arg His His Arg Arg Ser Arg Arg Arg Ser Gln
485 490 495

tct cca aac tgg ctg gag gag aaa cca ccc agc tac cgt tca ctg gat 1536
Ser Pro Asn Trp Leu Glu Glu Lys Pro Pro Ser Tyr Arg Ser Leu Asp
500 505 510

gtg act cca ggc aag aac aac atg aaa aag ggg aat gtg gag agg cgc 1584
Val Thr Pro Gly Lys Asn Asn Met Lys Lys Gly Asn Val Glu Arg Arg
515 520 525

ttg gtg agc ctg ggg cat cct gtg gag ggt cgg gca tga 1623
Leu Val Ser Leu Gly His Pro Val Glu Gly Arg Ala

530 535 540

<210> 4
<211> 540
<212> PRT
<213> Rattus sp.

<400> 4

Met Gly Ala Ser Met Gly Cys Gly Leu Leu Val Ala Gly Leu Leu Leu
1 5 10 15

Phe Thr Trp Leu Pro Ala Gly Cys Leu Ser Leu Leu Val Thr Val Gln
20 25 30

His Thr Glu Arg Tyr Val Thr Leu Phe Ala Ser Val Thr Leu Lys Cys
35 40 45

Asp Tyr Thr Thr Ser Ala Gln Leu Gln Asp Val Val Val Thr Trp Arg
50 55 60

Phe Lys Ser Phe Cys Lys Asp Pro Ile Phe Asp Tyr Phe Ser Ala Ser
65 70 75 80

Tyr Gln Ala Ala Leu Ser Leu Gly Gln Asp Pro Ser Asn Asp Cys Ser
85 90 95

Asp Asn Gln Arg Glu Val Arg Ile Val Ala Gln Arg Arg Gly Gln Ser
100 105 110

Glu Pro Val Leu Gly Val Asp Tyr Arg Gln Arg Lys Ile Thr Ile Gln
115 120 125

Asn Arg Ala Asp Leu Val Ile Asn Glu Val Met Trp Trp Asp His Gly
130 135 140

Val Tyr Tyr Cys Thr Ile Glu Ala Pro Gly Asp Thr Ser Gly Asp Pro
145 150 155 160

Asp Lys Glu Val Lys Leu Ile Val Leu His Trp Leu Thr Val Ile Phe
165 170 175

Ile Ile Leu Gly Ala Leu Leu Leu Leu Leu Leu Ile Gly Val Cys Trp
180 185 190

Cys Gln Cys Cys Pro Gln Tyr Cys Cys Cys Tyr Ile Arg Cys Pro Cys
195 200 205

Cys Pro Thr Cys Cys Cys Cys Pro Glu Glu Ala Leu Ala Arg His Arg
210 215 220

Tyr Met Lys Gln Val Gln Ala Leu Gly Pro Gln Met Met Glu Lys Pro
225 230 235 240

Leu Tyr Trp Gly Ala Asp Arg Ser Ser Gln Val Ser Ser Tyr Ala Met
245 250 255

Asn Pro Leu Leu Gln Arg Asp Leu Ser Leu Arg Ser Ser Leu Pro Gln
260 265 270

Met Pro Met Thr Gln Met Ala Ala His Pro Pro Val Ala Asn Gly Val
275 280 285

Leu Glu Tyr Leu Glu Lys Glu Leu Arg Asn Leu Asn Pro Ala Gln Pro
290 295 300

Leu Pro Pro Asp Leu Arg Thr Lys Ser Gly His Pro Cys Ser Met Leu
305 310 315 320

Ser Ser Leu Gly Ser Ala Glu Val Val Glu Arg Arg Val Ile His Leu
325 330 335

Pro Pro Leu Ile Arg Asp Pro Gln Pro Ser Arg Thr Ser Asn Ser Ser
340 345 350

His Gln Gln Arg Leu Asn Pro Val Pro Ser Arg Pro Trp Gly Pro Ser
355 360 365

Glu Gly Arg Arg Gln Arg Asn His Ser Asp Phe Leu Arg Glu Leu Gln
370 375 380

Asp Arg Gly Met Arg Pro Trp Ala Pro Gly Arg Gly Glu Leu Asp Pro
385 390 395 400

His Trp Ser Gly Arg His His Arg Ser Arg Pro Ser Glu Ser Ser Met
405 410 415

Pro Trp Ser Asp Trp Asp Ser Leu Ser Glu Cys Pro Ser Ser Ser Glu
420 425 430

Ala Pro Trp Pro Ser Arg Arg Pro Glu Pro Arg Glu Gly Ser Gln Arg
435 440 445

His Gly Arg Arg Arg His Arg Ser Tyr Ser Pro Pro Leu Pro Ser Gly
450 455 460

Pro Ser Ser Trp Ser Ser Glu Glu Glu Lys Glu Ser Leu Pro Arg Asn
465 470 475 480

Trp Gly Ala Gln Arg Arg His His Arg Arg Ser Arg Arg Arg Ser Gln
485 490 495

Ser Pro Asn Trp Leu Glu Glu Lys Pro Pro Ser Tyr Arg Ser Leu Asp
500 505 510

Val Thr Pro Gly Lys Asn Asn Met Lys Lys Gly Asn Val Glu Arg Arg
515 520 525

Leu Val Ser Leu Gly His Pro Val Glu Gly Arg Ala
530 535 540

<210> 5
<211> 18
<212> DNA
<213> Homo sapiens

<400> 5
atggcatggc ccaaactg 18

<210> 6
<211> 21
<212> DNA
<213> Homo sapiens

<400> 6
atcatgctga gcctcatttt c 21

<210> 7
<211> 20
<212> DNA
<213> Rattus sp.

<400> 7
atgggggcca gcatgggctg 20

<210> 8
<211> 21
<212> DNA
<213> Rattus sp.

<400> 8
tgcccgaccc tccacaggat g 21

<210> 9
<211> 20
<212> DNA
<213> Homo sapiens

<400> 9
tgctgctgat tggagtgtgc 20

<210> 10
<211> 20
<212> DNA
<213> Homo sapiens

<400> 10
gtctgggtca ttggcatctg 20

<210> 11
<211> 20
<212> DNA
<213> Rattus sp.

<400> 11
gagacaccac cgctctaggc 20

<210> 12
<211> 20
<212> DNA
<213> Rattus sp.

<400> 12
gacattcgct caggctgtcc 20

<210> 13
<211> 15
<212> DNA
<213> Rattus sp.

<400> 13
aagcaggcgg ccgag 15

<210> 14
<211> 21
<212> DNA
<213> Rattus sp.

<400> 14
atcaaaggtg gaagaatggg a 21

<210> 15
<211> 20
<212> DNA
<213> Rattus sp.

<400> 15
catggcttta ggcgaaccac 20

<210> 16
<211> 23
<212> DNA
<213> Rattus sp.

<400> 16
atctacattc ggcaggtatg gtc 23

<210> 17
<211> 19
<212> DNA
<213> Rattus sp.

<400> 17
tgctagccaa ccgtgcttc 19

<210> 18
<211> 20
<212> DNA
<213> Rattus sp.

<400> 18
ctcctcgtcc aggtacgcaa 20

<210> 19
<211> 21
<212> DNA
<213> Rattus sp.

<400> 19
aggtctctgt cacgcttctg g 21

<210> 20
<211> 19
<212> DNA
<213> Rattus sp.

<400> 20
ggctgagaca gcacgtgga 19

<210> 21
<211> 23
<212> DNA
<213> Homo sapiens

<400> 21
tgctgtgcta ggatccctcc acg 23

<210> 22
<211> 33
<212> DNA
<213> Artificial

<220>
<223> Description of Artificial Sequence: an artificially synthesized primer sequence

<400> 22
agaagcttgc cgcccccttt ctggcccttt tca 33

<210> 23
<211> 1525
<212> DNA
<213> Homo sapiens

<220>
<221> promoter
<222> (1)..(1525)
<223>

<400> 23
agatctagga caaggatgct cctctgggta gaggcgaaat gaaagatatc agcaacgcag 60

ggtaaggggc cccctcacag cagagcaggg catgacatgc gctcaggaga aaaaatatcg 120

acttctaagg ttagtgacca gaatgggagc ttcctgaaac tgtgtttaat gaatttaatg 180

aatttttatt catttatttt tcttgaagaa aaatgtgatg gctggtgggc ttcctgccgt 240

ggaaggagag actgagatta catgctacgt ggcctggtgc aaagagacct aaagtctttg 300

caaagcaaaa ggtcaaaacc ttgatctggc aaggtcaaaa ccttggtggg ggttgaggtc 360

tgtgagtggc ttctcagtga caatgaagga taagaaaaaa taaaataaat atgtacctac 420

ttataatatt tttataagaa aaaataaata aacattagaa tattagaata aataatagaa 480

taaataaata aatataccta cttgaacagt gcctagctat ggaaagctgc agcaggattt 540

ttttaaactc ccataacttt ttatttttct ataatatttt acttttatag ttgtagaaac 600

ttgagtcaac ttctctagtt tatctctacc ttctagaaac aaatatttat ttatttattt 660

atttatttat ttatttattt atttattttc gagaccgagt ttcgctctgt cgcccaggct 720

ggagtgcagt ggcgccatct ccgctcactt caacctccgc ctcccaggtt caagtaactc 780

tcgggcctca gcgtcccgag tagctgagat ttcaggggcg tgccaccacg cacggctgtt 840

ttgtattttt attagacagg gtttcgccat gtaggccagg ctggtctcga actcctgacc 900

tcggatgatc cacccgcctc ggcctcccaa agtgctggga ttacaggcgt gagccactgc 960

gcctggccta gatacgaatc tttatttcct agaaaatctg aacagacatt acgtgtaaac 1020

ttcatattac tgtgttttta gtaaaacaat gttttgcaag tatttataag ggaatggaaa 1080

tataacgcag gaaacacacc ctagaaagtt atatccacat aattctagaa ggagaggggg 1140

atacatttgc aggggatgag atttcccgag aacttctgga ggaaagtggg aatcttctcc 1200

gtcagcgggg tgggggacgg tgtttcagcg agcaggaggc ggcagcaggt agggaaggtg 1260

gccgcagtcc cccgggaggc gggggcggag caggaaacgg cggcgcgcgg ggcgggaggc 1320

ggagccgtgg ggagcgccgc aggtggggac gagccgggcg gcacctgccc cgggaccaga 1380

gcggacgctc cctccccgct gcgccgaggg aggggaaacc cgaggggttc cttggagaag 1440

gtggtgcgtc ctggggcggc agctgaggaa gaaagacgca gtgccccgaa gcccctgagc 1500

tgaaaagggc cagaaagggg gcggc 1525

<210> 24
<211> 30
<212> DNA
<213> Artificial

<220>
<223> Description of Artificial Sequence: an artificially synthesized primer sequence

<400> 24
agggatccat ggcatggccc aaactgcccg 30

<210> 25
<211> 30
<212> DNA
<213> Artificial

<220>
<223> Description of Artificial Sequence: an artificially

synthesized primer sequence

<400> 25
agctcgagta ggacgatgag ctttacttcc 30

## Description

Technical Field

This invention relates to a novel polypeptide relating to the improvement of chronic renal insufficiency and/or fibrotic conditions in the bladder, and a novel polynucleotide encoding said polypeptide. It also relates to the promoter of the aforementioned polypeptide, and a screening method which uses said promoter.

Background of the Invention

Organ fibrosis means a condition in which fibronectin, collagen and the like extracellular matrixes are excessively deposited to tissues, and such an excessive deposition of like extracellular matrixes is a morbid state of poor prognosis which induces an irreversible change of tissues and results in organ incompletion.

A large number of diseases which cause organ fibrosis are known, and particularly the fibrosis in the kidney is a histological chance that coincides with the advance of chronic renal insufficiency (c.f. Non-patent Reference 1, Non-patent Reference 2 and Non-patent Reference 3). That is, chronic renal insufficiency is a morbid state which causes glomerulosclerosis and fibrosis of uriniferous tubule and interstitium and results in the renal function extinction. Clinically, measurement of the glomerular filtration value (glomerular filtration rate: GFR) is used as one of the typical renal function evaluation means. Creatinine clearance is a most frequently used GFR inspection method, and the creatinine clearance can be guessed conveniently from the serum creatinine value. However, since creatinine is secreted from uriniferous tubule by the advance of glomerular disorder, it is known that the creatinine clearance shows a higher value compared to the true GFR, so that the actual decrease of GFR is underestimated. Also, since the kidney has a reserve ability, blood biochemical abnormal findings are not found in the initial step in which the renal function is lowered, namely under a condition in which nephron is lost but 50% or more of the same is maintained. The serum creatinine value is the same, and it is considered that when this shows an evidently abnormal value, 50% or more of the renal function is already lost (c.f. Non-patent Reference 3). In addition, when GFR is decreased to 30 to 40% or less independent of the original disease, a renal function disorder certainly advances at a certain ratio, and this renal function disorder advances even when activity of the original disease is evidently disappeared (c.f. Non-patent Reference 3). Accordingly, it is expected that treatment of the renal function reduction is started by finding it at a more early stage. Also, artificial dialysis is applied to an advanced renal function disorder, and renal transplantation is finally required (c.f. Non-patent Reference 3). Since artificial dialysis is continued life-long, each patient is forced to shoulder heavy physical and economical burdens. Thus, it has been considered that the necessity for the study regarding the analysis of cause of disease, healing and delaying of advance of chronic renal insufficiency is considerably high. In carrying out this study, a rat in which 5/6 of the kidney was extracted has been used as an experimental animal model mimicking chronic renal insufficiency. It is considered that a gene in which its expression is changing in the kidney of this rat has a high possibility of relating to chronic renal insufficiency (c.f. Non-patent Reference 4 and Non-patent Reference 5).

TGF-β (transforming growth factor-β) is already known as one of the factors which cause organ fibrosis. It is considered that organ fibrosis could be suppressed when the signal of overproduction extracellular matrix by this TGF-β is shut off, and actually, it was confirmed by an experimental animal that the neutralizing antibody of TGF-β suppresses renal interstitium fibrosis. However, since it has been revealed that TGF-β deletion animals die from multiple organ failure soon after birth, it is considered that an attempt for clinically applying suppression of TGF-β for a long period of time is still open to discussion (c.f. Non-patent Reference 1).

In addition, a medicament having an anti-fibrosis action for suppressing this over production of extracellular matrix as the main action has not been put on the market, so that it is the present situation that complete cure cannot be expected by a drug therapy of a morbid state in which organ fibrosis is advanced like the case of chronic renal insufficiency.

It has been reported that TGF-β accelerates expression of a fibronectin gene in a uriniferous tubule epithelial cell-derived NRK52E cell (Yokoi H. *et al., Am. J Physiol. Renal Physiol.:* 282, F 933 - F 942, 2002) and accelerates expression quantity of type I collagen (Creely J.J. *et al., Am. J Pathol.:* 140, 45 - 55, 1992).

Since about half the number of the cases of chronic renal insufficiency which results in the terminal stage renal insufficiency are originated from primary glomerulonephritis, it is considered that suppression of glomerulonephritis delays its advance to chronic renal insufficiency as a result. In recent years, it has been revealed that MCP-1 (monocyte chemotactic protein-1; monocyte chemotactic factor) is concerned in the advance of glomerulonephritis. That is, it has been reported that when a glomerulonephritis model (Umasugi glomerulonephritis) was prepared using an MCP-1 defective mouse, uriniferous tubule disorder of this animal was markedly improved (c.f. Non-patent Reference 6), and it has been reported that when antisense oligo of MCP-1 was administered to a Goodpasture syndrome model rat, infiltration of macrophage into interstitium was inhibited and renal function was maintained accompanied by the inhibition of MCP-1 gene expression (c.f. Non-patent Reference 7). Based on these results, a possibility has been suggested that inhibition of MCP-1 expression is effective in treating glomerulonephritis. As described in the foregoing, glomerulonephritis is the main original disease of chronic renal insufficiency, so that healing and advance delaying of this result in the delaying and prevention of its advance to chronic renal insufficiency as a result. Accordingly, it is considered that organ fibrosis can be prevented or delayed more strongly when not only the overproduction of extracellular matrix can be inhibited but also inflammatory reactions can be simultaneously inhibited, such as inhibition of MCP-1 gene expression. However, up to now, there is no medicament having such anti-fibrosis action and anti-inflammatory action.

The organ fibrosis is found in various organs such as the lungs, the trachea, the skin, the liver, the prostate, the pancreas, the bladder and the like (c.f. Non-patent Reference 1), and is not limited to the aforementioned kidney. For example, interstitial cystitis can be cited as one of the fibrotic conditions in the bladder (c.f. Non-patent Reference 8 and Non-patent Reference 9). The interstitial cystitis causes frequent urination, urinary urgency, pain in the bladder area and the like as the chief complaint, and definite cause of the disease is not clear. In the minor cases, they are apt to be mixed up with chronic cystitis, bladder neurosis, overactive bladder, prostatic hypertrophy and the like, and those which are differentially diagnosed have high seriousness. Based on these facts, decisive therapeutic method for interstitial cystitis has not been established (c.f. Non-patent Reference 9). Thus, so far there is no medicament applicable to interstitial cystitis, and antidepressants, anti-allergic drugs and the like are used in its treatment as a symptomatic therapy. At the terminal stage of interstitial cystitis, it must be rely on a surgical means, and it finally becomes an extremely invasive means such as bladder extraction and urinary diversion or application of new bladder formation (c.f. Non-patent Reference 9).

(Non-patent Reference 1)

Igaku-no Ayumi (Advance in Medical Science), vol. 201, no. 12 (Ishiyaku Shuppan, 2002)

(Non-patent Reference 2)

"Molecular Medicine", vol. 38, no. 8, Nakayama Shoten, 2001

(Non-patent Reference 3)

Saishin Naikagaku Taikei Jin Hinyoki Shikkan 4 Jinfuzen (New Internal Medicine Kidney and Urinary Organ Diseases 4 Renal Insufficiency), Nakayama Shoten, 1995

(Non-patent Reference 4)

"The American Journal of Pathology", (USA), 1975, vol. 79, pp. 95 - 106

(Non-patent Reference 5)

Jin to Toseki (Kidney and Dialysis), vol. 31, supplement "Jin Shikkan Model (Renal Disease Model)", Tokyo Igaku-sha, 1991

(Non-patent Reference 6)

"The Journal of Clinical Investigation", (USA), 1999, vol. 103, pp. 73 - 80

(Non-patent Reference 7)

"Kidney International", (USA), 2000, vol. 57, pp. 927 - 936

(Non-patent Reference 8)

Dai V Ban Essential Hinyoki Kagaku (Fifth Edition Essential Urinary Organ Science), Ishiyaku Shuppan, 1989

(Non-patent Reference 9)

Kanshitsu-sei Bokoen -Ekigaku kara Chiryo made- (Interstitial Cystitis -from Epidemiology to Treatment-), edited by Japanese Society for Interstitial Cystitis, Igaku Tosho Shuppan, 2002

Disclosure of the Invention

As a result of repeating intensive studies, the present inventors have succeeded in cloning human and rat derived cDNA of a novel nucleotide sequence coding for a protein FREP (fibrosis related protein) which is useful in diagnosing chronic renal insufficiency and fibrotic conditions of the bladder and is useful in improving and/or preventing organ fibrosis. It was revealed that expression quantity of the aforementioned rat gene is markedly decreased in the kidney of a chronic renal insufficiency model rat, compared with a normal individual, thereby rendering possible an inspection method useful in diagnosing chronic renal insufficiency morbid states from the expression quantity of the gene. Also, it was revealed that the expression quantity is markedly decreased in the bladder of a rat suffering from the induction of fibrotic conditions in the bladder, compared with a normal individual, thereby rendering possible an inspection method useful in diagnosing fibrotic conditions of the bladder from the expression quantity of the gene. Further, it was revealed that due to the overproduction of FREP, the production of extracellular matrixes such as fibronectin and type I collagen, wherein it is known that their overproduction becomes the cause of fibrotic conditions, is suppressed, and the production of MCP-1 which is a factor relating to the advance of glomerulonephritis is also suppressed. Also, by obtaining an N-terminal moiety protein of FREP (FREP-N; from 1 st to 166th positions of the amino acid sequence represented by SEQ ID NO:2), it was revealed that the production of fibronectin and type I collagen is suppressed by said protein. Based on these findings, it was revealed that FREP or a part thereof is useful in improving and/or preventing organ fibrosis and inflammation.
In addition, the promoter region of the FREP gene was identified, and a method for screening a substance useful in treating chronic renal insufficiency and/or fibrotic conditions of the bladder was constructed and provided, thereby accomplishing the invention.
That is, the invention relates to

[1] a polypeptide which comprises the amino acid sequence represented by SEQ ID NO:2 or SEQ ID NO:4 and which suppresses expression of type I collagen, fibronectin and/or MCP-1, or a polypeptide which comprises an amino acid sequence represented by SEQ ID NO:2 or SEQ ID NO:4 wherein from 1 to 10 amino acids of the amino acid sequence are deleted, substituted and/or inserted and which suppresses expression of type I collagen, fibronectin and/or MCP-1,
[2] a polypeptide which comprises the amino acid sequence represented by SEQ ID NO:2 or SEQ ID NO:4,
[3] a polypeptide which comprises an amino acid sequence represented by the 1st to 166th positions of the amino acid sequence represented by SEQ ID NO:2, or a polypeptide which comprises an amino acid sequence represented by the 1st to 166th positions of the amino acid sequence represented by SEQ ID NO:2 wherein from 1 to 10 amino acids of an amino acid sequence are deleted, substituted and/or inserted and which suppresses expression of type I collagen, fibronectin and/or MCP-1,
[4] a polynucleotide which encodes the polypeptide described in [1] to [3],
[5] an expression vector which comprises the polynucleotide described in [4],
[6] a cell transfected with the expression vector described in [5],
[7] a medicinal composition for improving and/or preventing fibrosis, which contains a polypeptide described in any one of the following (a) to (c) as the active ingredient;

(a) a polypeptide which comprises the amino acid sequence represented by SEQ ID NO:2, by the 1st to 166th positions of the amino acid sequence represented by SEQ ID NO:2, or by SEQ ID NO:4, and which suppresses expression of type I collagen, fibronectin and/or MCP-1,
(b) a polypeptide which comprises an amino acid sequence represented by SEQ ID NO:2, by the 1st to 166th positions of the amino acid sequence represented by SEQ ID NO:2, or by SEQ ID NO:4, wherein from 1 to 10 amino acids of the amino acid sequence are deleted, substituted and/or inserted, and which suppresses expression of type I collagen, fibronectin and/or MCP-1, and
(c) a polypeptide which comprises the amino acid sequence represented by SEQ ID NO:2, by the 1st to 166th positions of the amino acid sequence represented by SEQ ID NO:2, or by SEQ ID NO:4,

[8] a medicinal composition for improving and/or preventing fibrosis, which contains a polynucleotide coding for the polypeptide described in [7] as the active ingredient,
[9] a polynucleotide which comprises (1) the nucleotide sequence represented by SEQ ID NO:23, or (2) a nucleotide sequence represented by the 1124th to 1525th positions of the nucleotide sequence represented by SEQ ID NO:23,
[10] an expression vector which comprises the polynucleotide described in [9],
[11] a cell transfected with the expression vector described in [10],

[12] a tool for screening a fibrosis improving agent, comprising (1) a polynucleotide which comprises the polynucleotide described in [9] or a nucleotide sequence represented by the 1124th to 1525th positions of the nucleotide sequence represented by SEQ ID NO:23 wherein from 1 to 10 bases of the nucleotide sequence are deleted, substituted and/or inserted, and which has promoter activity of the polynucleotide described in [4], (2) an expression vector comprising the polynucleotide described in (1), or (3) a cell transfected with the aforementioned expression vector,

[13] use of (1) a polynucleotide which comprises the polynucleotide described in [9] or a nucleotide sequence represented by the 1124th to 1525th positions of the nucleotide sequence represented by SEQ ID NO:23 wherein from 1 to 10 bases of the nucleotide sequence are deleted, substituted and/or inserted, and which has promoter activity of the polynucleotide described in [4], (2) an expression vector comprising the polynucleotide described in (1), or (3) a cell transfected with the aforementioned expression vector,
for screening a fibrosis improving agent,

[14] a method for screening a fibrosis improving agent, characterized in that it comprises

(1) a step for allowing a cell transfected with an expression vector containing a polynucleotide which comprises (i) the polynucleotide described in [9] or (ii) a nucleotide sequence represented by 1124th to 1525th positions of the nucleotide sequence represented by SEQ ID NO:23 wherein from 1 to 10 bases of the nucleotide sequence are deleted, substituted and/or inserted, and which has promoter activity of the polynucleotide described in [4], to contact with a substance to be tested,
(2)a step for detecting the promoter activity, and
(3) a step for selecting a substance which accelerates the promoter activity, and

[15] a method for producing a medical composition for improving fibrotic conditions, characterized in that it comprises
a step for carrying out screening using the screening method described in [14], and
a step for preparing pharmaceutical preparations.

Nothing is known about the sequences identical to the polypeptide of the invention described in SEQ ID NO: 2, the 1st to 166th positions of SEQ ID NO:2, or SEQ ID NO:4 and polynucleotides which encode the same. U.S. Patent Laying Open of Application 2002/090672 specification, and International Publication 01/55315 pamphlet, 01/54474 pamphlet and 01/90304 pamphlet disclose a large number of sequences including sequences having homology with partial sequences of the polypeptide or polynucleotide of the invention and describe that the disclosed novel polypeptides and the like are useful for the diagnosis and prevention of diseases or screening of agousts or antagonists. However, there are no illustrative descriptions on the use of the sequences having homology with the polypeptide or polynucleotide of the invention, and there are no descriptions also on the ground of the described uses of the large number of sequences. In addition, there is no information that these polypeptides were actually obtained or illustrative information on how to obtain them. What is more, when descriptions of Examples are examined, they are merely described in the present tense, and there is absolutely no proof about their use.
After the priority date of the instant application, sequences containing the 1 st to 166th positions of the amino acid sequence represented by SEQ ID NO:2 as one of the polypeptides of the invention were disclosed as accession numbers BC044240 and AY134857 in a sequence data base GenPept, and sequences having homology with partial sequences of the amino acid sequence represented by SEQ ID NO:2 as one of the polypeptides of the invention were disclosed as accession numbers AK035504, BC010485, AK036844 and BC057644, but this is merely a disclosure of sequences and there is no description on their illustrative use. In addition, International Publication 03/025130 pamphlet laid open after the priority date of the instant application describes a polypeptide (human REMAP-6) consisting of 546 amino acids which coincide with the 1st to 533rd positions of the amino acid sequence (635 amino acids) represented by SEQ ID NO:2 as one of the polypeptides of the invention (namely, a sequence comprising the 1 st to 166th positions of the amino acid sequence represented by SEQ ID NO:2 as one of the polypeptides of the invention). A large number of diseases, in which several REMAPs including human REMAP-6 are considered to be concerned, are enumerated over several pages of the pamphlet, and fibrosis is included therein. However, when descriptions of Examples are examined, they are merely described in the present tense, and there is absolutely no proof about their use.
The present inventors have found the polypeptide and polynucleotide of the invention for the first time and revealed for the first time that they are useful for the diagnosis of fibrotic conditions and useful in improving and/or preventing fibrosis and inflammation. In addition, the method for screening a substance useful in treating chronic renal insufficiency and/or fibrotic conditions of the bladder, which uses the promoter region of FREP gene, is a method provided for the first time by the inventors.

Brief Description of the Drawings

Fig. 1 is a graph showing expression of human FREP in cultured cells. MWM represents molecular weight marker. Lane 1 shows a sample in which pcDNA3-hFREP was introduced, and lane 2 a sample in which pcDNA3.1 was introduced, and lane 3 a COS-1 cell sample in which nothing was introduced.
Fig. 2 is a graph showing a comparison of FREP expression quantities in a chronic renal insufficiency model rat (5/6 kidney extracted rat) and a normal rat. The axis of ordinate of the drawing shows relative amount of mRNA, lane 1 shows a result of fake operation (2 weeks), and lanes show 2 to 4 results of 5/6 kidney extraction (lane 2; 2 weeks, lane 3; 4 weeks, lane 4; 6 weeks).
Fig. 3 is a graph showing changes in the fibronectin expression quantity in a human FREP stable expression cell and an FREP non-introduced cell. The axis of ordinate in the drawing shows relative amount of mRNA, and the axis of abscissa shows amount of TGF-β. The oblique line bar shows a result of using a pcDNA introduced cell, and the black bar that of using a human FREP stable expression cell.
Fig. 4 is a graph showing changes in the type I collagen expression quantity in the human FREP stable expression cell and FREP non-introduced cell. The axis of ordinate in the drawing shows relative amount of mRNA, and the axis of abscissa shows amount of TGF-β. The oblique line bar shows a result of using he pcDNA introduced cell, and the black bar that of using the human FREP stable expression cell.
Fig. 5 is a graph showing changes in the MCP-1 expression quantity by BSA in the human FREP stable expression cell and FREP non-introduced cell. The axis of ordinate in the drawing shows relative amount of mRNA, and the axis of abscissa shows amount of BSA. The oblique line bar shows a result of using the pcDNA introduced cell, and the black bar that of using the human FREP stable expression cell.
Fig. 6 is a graph showing the luciferase activity measured in Example 8. The axis of ordinate in the drawing shows relative luciferase activity, and 1 of the axis of abscissa shows a result of using pGL3-Basic, and 2 of the same shows that of pGL3-FD-402bp, and 3 of the same shows that of pGL3-FR-1525bp.
Fig. 7 is a graph showing purified FREP-N protein. MWM shows molecular weight marker. Lane 1 shows passed-trough fraction, lanes 2 and 3 show washed fractions, and lanes 4,5 and 6 show eluted fractions.

Best Mode for Carrying Out the Invention

The following describes the invention in detail. Unless otherwise noted, the gene manipulation techniques in this description can be carried out in accordance with known techniques such as of "Molecular Cloning" Sambrook, J *et al,* Cold spring Harbor Laboratory Press, 1989, and unless otherwise noted, the protein manipulation techniques can be carried out in accordance with known techniques such as of "Tanpaku Jikken Protocol (Protein Experimentation Protocol)" (Shuhjun-sha, 1997).

<Polypeptide of the invention>

The polypeptide of the invention includes

(1) a polypeptide consisting of the amino acid sequence represented by SEQ ID NO:2, by the 1 st to 166th positions of the amino acid sequence represented by SEQ ID NO:2, or by SEQ ID NO:4; and
(2) i) a polypeptide which comprises the amino acid sequence represented by SEQ ID NO:2 or SEQ ID NO:4 and which suppresses expression of type I collagen, fibronectin and/or MCP-1, ii) a polypeptide which comprises an amino acid sequence represented by SEQ ID NO:2 or SEQ ID NO:4 wherein from 1 to 10 (preferably from 1 to 7, more preferably from 1 to 5, further preferably from 1 to 3) amino acids are deleted, substituted and/or inserted, and which suppresses expression of type I collagen, fibronectin and/or MCP-1, or iii) a polypeptide which comprises an amino acid sequence represented by the 1st to 166th positions of the amino acid sequence represented by SEQ ID NO:2 wherein from 1 to 10 (preferably from 1 to 7, more preferably from 1 to 5, further preferably from 1 to 3) amino acids are deleted, substituted and/or inserted, and which suppresses expression of type I collagen, fibronectin and/or MCP-1 (to be referred to as functionally equivalent modified product hereinafter).

The polypeptide of the invention is not limited to human- and rat-derived polypeptides with the proviso that they correspond to either one of the aforementioned (1) and (2), and other vertebral animals(e.g., mouse, rabbit, horse, sheep, dog, monkey, cat, bear, pig, domestic fowl and the like) are also included therein. In addition, they are is not limited to natural polypeptides with the proviso that they correspond to either one of the aforementioned (1) and (2), and artificially produced mutants are also included therein.

The term "suppresses expression of type I collagen, fibronectin and/or MCP-1" means that the expression of type I collagen, fibronectin and/or MCP-1 is suppressed by the presence of the polypeptide of interest, and whether or not the polypeptide to be examined "suppresses expression of type I collagen, fibronectin and/or MCP-1" can be verified by expressing the polypeptide to be examined in a cell in which type I collagen, fibronectin and/or MCP-1 is expressed, and examining expressed quantity of type I collagen, fibronectin and/or MCP-1. For example, it can be verified by the method of Example 6 (type I collagen and fibronectin), Example 7 (MCP-1) or Example 10 (type I collagen and fibronectin). Preferred as the polypeptide of the invention is a polypeptide in which, in the method of Example 6, 10% or more (preferably 20% or more, more preferably 30% or more) of the expressed quantity of fibronectin is decreased by effecting expression of the polypeptide to be examined under a TGF-β un-added condition and 10% or more (preferably 20% or more, more preferably 30% or more, further preferably 40% of more) of the expressed quantity of fibronectin is decreased under a TGF-β added condition. Alternatively, preferred as the polypeptide of the invention is a polypeptide in which, in the method of Example 6, 10% or more (preferably 20%, more preferably 40%, further preferably 60%, particularly preferably 80% or more) of the expressed quantity of type I collagen is decreased by effecting expression of the polypeptide to be examined under a TGF-β un-added or added condition. A polypeptide which suppresses expression of all of the type I collagen, fibronectin and MCP-1 is most desirable as the polypeptide of the invention.
The polypeptide described in SEQ ID NO:4 as one of the polypeptides of the invention is a polypeptide in which its expression quantity is greatly changed by a normal condition and a fibrotic condition of an organ in kidney tissue and/or bladder tissue and its expressed quantity is decreased in the morbid state. Accordingly, chronic renal insufficiency and/or fibrotic conditions of the bladder is diagnosed by measuring amount of the polypeptide of the invention. The polypeptide of the invention can be used for the preparation of an antibody capable of specifically recognizing the polypeptide of the invention, and said antibody is useful in diagnosing chronic renal insufficiency and/or fibrotic conditions of the bladder. In addition, since the polypeptide consisting of the amino acids represented by SEQ ID NO:2 as one of the polypeptides of the invention or its 1 st to 166th position polypeptide suppressed overproduction of extracellular matrixes such as fibronectin and type I collagen, wherein it is known that their overproduction becomes the cause of fibrotic conditions, and also suppressed production of MCP-1 which is a factor relating to the advance of glomerulonephritis, the polypeptide of the invention is useful as a fibrosis improving agent.
The polypeptide consisting of the amino acids represented by SEQ ID NO:2 is a human FREP protein, and the polypeptide consisting of the amino acids represented by SEQ ID NO:4 is a rat FREP protein.
As the polypeptide of the invention, a polypeptide in which an appropriate marker sequence is added to the N-terminal and/or C-terminal of the human FREP protein or rat FREP protein is included. As the aforementioned marker sequence, a sequence for easily carrying out expression verification, purification or the like of polypeptide can be used, and its examples include FLAG epitope, hexa-histidine tag, hemagglutinin tag, myc epitope and the like.
Most desirable as the polypeptide of the invention is a polypeptide consisting of the amino acid sequence represented by SEQ ID NO:2, by the 1st to 166th positions of SEQ ID NO:2, or by SEQ ID NO:4.

<Polynucleotide of the invention>

Included in the polynucleotide of the invention are

[1] a polynucleotide encoding the polypeptide of the invention and
[2] a polynucleotide consisting of (1) the nucleotide sequence represented by SEQ ID NO:23, or (2) a nucleotide sequence represented by the 1124th to 1525th positions of the nucleotide sequence represented by SEQ ID NO: 23 (to be referred to as promoter type polynucleotide of the invention hereinafter).

The polynucleotide coding for the polypeptide of the invention may be derived from any species with the proviso that it is a polypeptide consisting of the amino acid sequence represented by SEQ ID NO:2, by the 1 st to 166th positions of SEQ ID NO:2, or by SEQ ID NO:4, or a nucleotide sequence coding for a functionally equivalent modified product. Preferred is a polynucleotide consisting of a nucleotide sequence coding for the amino acid sequence represented by SEQ ID NO:2, by the 1 st to 166th positions of SEQ ID NO:2, or by SEQ ID NO:4, and more preferred is a nucleotide sequence represented by SEQ ID NO:1, by the 1st to 498th positions of SEQ ID NO:1, or by SEQ ID NO: 3. In this connection, both of DNA and RNA are included in the "polynucleotide" of this description.
The polynucleotide coding for the polypeptide of the invention can include all mutants so far as they encode the polypeptide of the invention. More illustratively, it can include naturally existing allele mutants and mutants which do not exist in the nature. The aforementioned mutants may be generated in the nature by mutation, but they can be prepared by an artificial modification. All of the mutant genes coding for the aforementioned polypeptide of the invention are included therein disregard of the cause and means of the aforementioned polynucleotide mutation. Examples of the artificial means for preparing aforementioned mutants include the base specific substitution method (Methods in Enzymology, (1987) 154, 350, 367 - 382) and the like genetic engineering techniques, as well as the phosphoric acid triester method, phosphoric acid amidide method and the like chemical synthesis means (Science (1968) 150, 178). It is possible to obtain DNA having a desired base substitution by a combination of them. Alternatively, it is possible to effect generation of substitution to a nonspecific base in a DNA molecule by a repetition work of PCR method, or by allowing manganese ion and the like in the reaction liquid.

The polynucleotide polypeptide of the invention can be easily produced and obtained by general genetic engineering techniques based on the sequence information disclosed by the invention.

The polynucleotide of the invention obtained for example in the following manner, but without limiting to this method, it can also be obtained by conventionally known operations "Molecular Cloning" [Sambrook, J *et al.,* Cold Spring Harbor Laboratory Press, 1989 and the like].

For example, (1) a method which uses PCR, (2) a method which uses generally used genetic engineering techniques (namely, a method in which a transformant containing desired amino acids is selected from transformants transformed with a cDNA library), (3) a chemical synthesis method and the like can be cited. Regarding each of the production methods, it can be carried out in the same manner as described in WO 01/34785.

Regarding the method which uses PCR, for example, the polynucleotide described in this description can be produced by the procedure of "Mode for Carrying Out the Invention", 1) Production method of protein gene, a) First production method, described in the aforementioned patent reference. Human kidney can for example be cited as the "human cell or tissue having the ability to produce the protein of the invention" in said description. A first strand cDNA can be synthesized by extracting mRNA from human kidney and carrying out reverse transcriptase reaction of this mRNA in the presence of a random primer or oligo dT primer. The polynucleotide of the invention or a part thereof can be obtained using the thus obtained first strand cDNA by subjecting it to polymerase chain reaction (PCR) using two primers interposing a partial region of the gene of interest. More illustratively, the polynucleotide of the invention can be produced, for example, by the method described in Example 1.

Regarding the method which uses generally used genetic engineering techniques, for example, the polynucleotide of the invention can be produced by the procedure of "Mode for Carrying Out the Invention", 1) Production method of protein gene, b) Second production method, described in the aforementioned patent reference.

Regarding the method which uses chemical synthesis, for example, the polynucleotide of the invention can be produced by the procedure of "Mode for Carrying Out the Invention", 1) Production method of protein gene, c) Third production method and d) Fourth production method, described in the aforementioned patent reference.

Among the polynucleotides of the invention obtained in this manner, making use of a part or entire portion of nucleotide sequence of a polynucleotide coding for the polypeptide of the invention, expression level of the polynucleotide coding for the polypeptide of the invention can be specifically detected in individual or various tissues.

AS such a detection method, RT-PCR (reverse transcription-polymerase chain reaction), northern blotting analysis*, in situ* hybridization and the like methods can be exemplified. The primer which is used when a polynucleotide coding for the polypeptide of the invention is detected by RT-PCR is not particularly limited, with the proviso that it can specifically amplify said polynucleotide alone, and can be optionally set based on the sequence information of the polynucleotide coding for the polypeptide of the invention. A primer which can specifically amplify a polynucleotide coding for the polypeptide of the invention can be used as a specific primer or specific probe for detecting a polynucleotide coding for the polypeptide of the invention.

In addition, the polynucleotide coding for the polypeptide of the invention can also be used for producing the polypeptide of the invention in the body by gene therapy.

A substance capable of accelerating expression of the polypeptide of the invention can be screened by analyzing whether or not a test compound accelerates the promoter activity of the invention using the promoter type polynucleotide of the invention. The present inventors have revealed that, when FREP as one of the polypeptides of the invention is over-expressed, expression of fibronectin and type I collagen, known to be a cause of fibrotic conditions from uriniferous tubule epithelial cells, and their expression acceleration by TGF-β are suppressed (Example 6), acceleration of the expression quantity of MCP-1, which is related to the advance of glomerulonephritis from uriniferous tubule epithelial cells, by BSA is suppressed (Example 7), and expression of fibronectin and type I collagen is suppressed in the presence of FREP (a protein consisting of 1st to 166th position amino acids of the amino acid sequence represented by SEQ ID NO:2) (Example 10). Based on these facts, the aforementioned substance which accelerates expression of the polypeptide of the invention is useful as an agent for improving and/or preventing fibrotic conditions. Thus, the promoter type polynucleotide of the invention can be used as a screening of an agent for improving and/or preventing fibrotic conditions.

<Production methods of the expression vector, cell and polypeptide of the invention>

A method for producing the polypeptide of the invention, characterized in that the transfected cell of the invention is cultured, is also included in the invention.

The polynucleotide coding for the polypeptide of the invention, obtained in the aforementioned manner, can be used in effecting expression of the polypeptide of the invention in a test tube or in a test cell, by connecting it to the downstream of an appropriate promoter by the method described in "Molecular Cloning" [Sambrook, J *et al,* Cold spring Harbor Laboratory Press, 1989] or the like.

Illustratively, when a polynucleotide containing a specified promoter sequence is added to the upstream of the initiation codon for the polypeptide of the invention, existing by the 5' side of the polynucleotide coding for the polypeptide of the invention obtained in the aforementioned manner, it becomes possible to effect expression of the polypeptide of the invention by the transcription and translation of the gene in a cell-free system which uses this as the template.

Alternatively, when the aforementioned polynucleotide coding for the polypeptide of the invention is integrated into an appropriate vector plasmid and introduced as a form of plasmid into a host cell, expression of the polypeptide of the invention inside the cell becomes possible. Alternatively, a cell in which such a construction is integrated into chromosomal DNA may be obtained and used. More illustratively, a fragment containing an isolated polynucleotide can transfect host cells of an eucaryote or prokaryote by again integrating it into an appropriate vector plasmid. Further, by introducing an appropriate promoter and a sequence concerned in the gene expression into these vectors, it is possible to effect expression of the polypeptide of the invention in respective host cells.

An expression vector for producing the polypeptide of the invention and an expression vector for producing the polypeptide of the invention in a body by gene therapy are included in the expression vector of the invention.

As the expression vector for vertebral animal cells, those which generally have a promoter locating at the upstream of a polynucleotide to be expressed, a splice site of RNA, a polyadenylation site, a transcription termination sequence and the like can be used, and they can have a replication origin as occasion demands. As an example of the aforementioned expression vector, for example, pSV2dhfr having SV40 early promoter (Subramani, S. *et al., Mol. Cell. Biol.,* 1, 854 - 864, 1981), pEF-BOS having human elongation factor promoter (Mizushima, S. and Nagata, S., *Nucleic Acids Res.,* 18, 5322, 1990), pCEP4 having cytomegalovirus promoter (Invitrogen) or the like can be cited.

When COS cell is used as the host cell, those which have the SV40 replication origin, can perform autonomous replication in the COS cell, and further have a transcription promoter, a translation termination signal and an RNA splicing site can be used as the expression vector, and their examples include pME18S (Maruyama, K. and Takebe, Y., *Med. Immunol.,* 20, 27 - 32, 1990), pEF-BOS (Mizushima, S. and Nagata, *S., Nucleic Acids Res.,* 18, 5322, 1990), pCDM8 (Seed, B., Nature, 329, 840 - 842, 1987) and the like.

The aforementioned expression vector can be incorporated into COS cell by, for example, the DEAE-dextran method (Luthman, H. and Mugnusson, G., *Nucleic acids Res.,* 11, 1295 - 1308, 1983), the calcium phosphate-DNA coprecipitation method (Graham, F.L. and van der Ed, A.J., *Virology,* 52, 456 - 457, 1973), a commercially available transfection reagent (e.g., FuGENE™6 Transfection Reagent; mfd. by Roche Diagnostics), the electroporation method (Neumann, E. *et al., EMBO J.,* 1, 841 - 845, 1982) or the like.

Also, when CHO cell is used as the host cell, a transformed cell which can stably produce the polypeptide of the invention can be obtained by co-transfecting a vector capable of expressing a neo gene that functions as a G418 resistance marker, such as pRSVneo (Sambrook, J. *et al,* "Molecular Cloning - A Laboratory Manual", Cold spring Harbor Laboratory, NY, 1989), pSV2-neo (Southern, P.J. and Berg, P., *J. Mol. Appl. Genet.,* 1, 327 - 341, 1982) or the like, together with an expression vector containing a polynucleotide coding for the polypeptide of the invention, and selecting a G418-resistant colony.

In addition, when 293-EBNA cell is used as the host cell, pCEP4 (Invitrogen) which has the Epstein-Barr virus replication origin and can perform autonomous replication in the 293-EBNA cell, or the like, can be used as the expression vector.

As the vector for gene therapy, for example, there are (1) virus vectors or (2) non-virus vectors, and a generally used vector (e.g., retrovirus, adenovirus, Sendai virus or the like in the case of a virus vector) can be used [Jikken Igaku (Experimental Medical Science) (Supplement), edited by F. Takahisa, "Idenshi Chiryo no Saizensen (Frontier of Gene Therapy)", vol. 12, no. 15, 1994].

The host cell is not particularly limited and may be any cell which can detect expressed amount of the polypeptide of the invention at a messenger RNA level or protein level. It is more desirable to use a kidney derived cell or bladder derived cell in which intrinsic FREP is abundantly present, as the host cell.

In addition, a vector which can effect expression of the promoter type polynucleotide of the invention is also included in the expression vector of the invention.

The promoter expression cell of the invention can be produced by integrating the promoter type polynucleotide of the invention into a host cell optionally selected in response to the purpose. It is desirable to produce it by integrating the promoter type polynucleotide of the invention into a vector optionally selected in response to the purpose; for example, when the purpose is to construct a system for analyzing whether or not it accelerates the promoter activity, it is desirable to produce it by integrating the promoter type polynucleotide of the invention into a vector integrated with a reporter gene such as of luciferase or the like as shown in Example 8. The reporter gene to be fused with the promoter region is not particularly limited with the proviso that it is generally used, but an enzyme gene or the like whose quantitative measurement can be easily made is desirable. For example, a bacterial transposon derived chloramphenicol acetyltransferase gene (CAT), a firefly derived luciferase gene (Luc), a jellyfish derived green fluorescent protein gene (GFP) and the like can be cited. The reporter gene should be fused functionally with the promoter type polynucleotide of the invention.

For example, when the purpose is to construct a screening system for a substance which controls the promoter activity of the invention, it is desirable to use a mammal (e.g., human, mouse, rat or the like) derived cell as the cell, and more desirable to use a human derived cell.

The expression vector and cell which contain the promoter type polynucleotide of the invention can be used in constructing the screening system for a substance which controls the promoter activity of the invention shown in Example 8, and are useful tools of said screening.

The method for expressing a gene by transfecting a host cell can be carried out, for example, by the method described in the "Mode for Carrying Out the Invention", 2) Production methods of vector of the invention, host cell of the invention and recombinant protein of the invention, of the aforementioned patent reference. The cell of the invention can be obtained, for example, by transfecting a desired host cell by the aforementioned expression vector. More illustratively, for example, an expression vector of a desired protein can be obtained by integrating a desired polynucleotide into an expression vector pcDNA3.1 for mammal cell use as described in Example 2, and the transformed cell of the invention can be produced by incorporating said expression vector into COS-1 cell using the lipofection method.

The desired transformed cell obtained in the above can be cultured in accordance with a usual method, and the desired protein is produced by said culturing. As the medium to be used in said culturing, various generally used ones can be optionally selected in response to the employed host cell. For example, in the case of the aforementioned COS-1 cell, Dulbecco's modified Eagle's minimum essential medium (DMEM) supplemented with fetal bovine serum (FBS) or the like serum component can be used by further adding G418 thereto. As the cell transfected with an expression vector containing a polynucleotide coding for the polypeptide of the invention, a cell expressing the polypeptide of the invention is desirable.

By culturing the cell of the invention, the polypeptide of the invention produced in the cell can be detected, determined and further purified. For example, it is possible to detect and purify the polypeptide of the invention by a western blotting method or an immunoprecipitation method using an antibody capable of binding to the polypeptide of the invention. Alternatively, by effecting expression of the polypeptide of the invention as a fusion protein with glutathione S-transferase (GST), protein A, β-galactosidase, maltose-binding protein (MBP) or the like appropriate tag protein, the polypeptide of the invention can be detected by a western blotting method or immunoprecipitation method using an antibody specific for such a tag protein, and can be purified making use of the tag protein. More illustratively, it can be purified making use of a tag protein in the following manner.

The polypeptide of the invention (e.g., a polypeptide represented by SEQ ID NO:2, the 1st to 166th positions of SEQ ID NO:2 or SEQ ID NO:4) can be obtained by expressing it in a cultured cell through the integration of a polynucleotide coding for the polypeptide of the invention (e.g., a polynucleotide represented by SEQ ID NO:1, the 1st to 498th positions of SEQ ID NO:1 or SEQ ID NO:3) into, for example, a vector with which His tag is fused, more illustratively, for example, the pcDNA3.1/V5-His-TOPO (Invitrogen) or the like described in Example 1, purifying it using His tag, and then removing the tag moiety. For example, each of the human and rat FREP expression plasmids prepared using pcDNA3.1/V5-His-TOPO in Example 1 is designed in such a manner that V5 and His tag are added to the C-terminal of FREP. Based on this, the FREP protein can be purified from the PREP-expressed cultured cell shown in Example 2, making use of the His tag. Illustratively, the FREP protein fused with His tag can be isolated and purified from an extract of disrupted cells by binding it to a HisTrap column (Amersham Bioscience), in accordance with a conventionally known method (Jikken Igaku Bessatsu (Experimental Medical Science, Supplement), Tanpakushitsu no Bunshikan Sogo Sayo Jikken Ho (Experimentation method on intermolecular interaction of protein), Nakahara *et al.,* p. 32, 1996) or Example 10. More illustratively, the polypeptide expression cells of the invention cultured in a culture flask (e.g., a Petri dish of 10 cm in diameter) are scraped off by adding an appropriate amount of a buffer (e.g., 1 ml), disrupted and then centrifuged at 15000 revolution per minutes for 5 minutes, and the separated supernatant is linked the HisTrap column. The polypeptide of the invention can be purified by washing the column with an appropriate buffer and then eluting and fractionating it with the buffer containing imidazole in a high concentration. As the aforementioned buffer, a phosphate buffer (20 mM sodium phosphate, 0.5 M sodium chloride, pH 7.4) can for example be used. His tag in the purified protein molecule can be removed from the molecule, for example, by designing in such a manner that His tag is fused to the N-terminal side and using TAGZyme System (Qiagen).

Alternatively as occasion demands, it can also be purified by a method which does not use a tag protein, such as various separation operations that use physical properties and chemical properties of the polypeptide of the invention. Illustratively, the use of ultrafiltration, centrifugation, gel filtration, adsorption chromatography, ion exchange chromatography, affinity chromatography and high performance liquid chromatography can be exemplified.

The polypeptide of the invention can be produced by a general chemical synthesis method in accordance with the amino acid sequence information shown in SEQ ID NO:2. Illustratively, it includes peptide synthesis methods by liquid phase and solid phase methods. The synthesis may be carried out by binding amino acids one by one in succession or synthesizing peptide fragments consisting of several amino acids and then binding them. Purification of the polypeptide of the invention obtained by these means can be carried out in accordance with the aforementioned various methods.

<Medicinal composition of the invention for improving and/or preventing fibrosis>

The inventors have shown in Examples 6, 7 and 10 that FREP or FREP-N as one of the polypeptides of the invention suppresses type I collagen, fibronectin and/or MCP-1. Based on these findings,

(a) a polypeptide which comprises the amino acid sequence represented by SEQ ID NO:2, by the 1st to 166th positions of the amino acid sequence represented by SEQ ID NO:2, or by SEQ ID NO:4, and which suppresses expression of type I collagen, fibronectin and/or MCP-1,
(b) a polypeptide which comprises an amino acid sequence represented by SEQ ID NO:2, by the 1st to 166th positions of the amino acid sequence represented by SEQ ID NO:2, or by SEQ ID NO:4, wherein from 1 to 10 amino acids of the amino acid sequence are deleted, substituted and/or inserted, and which suppresses expression of type I collagen, fibronectin and/or MCP-1, and
(c) a polypeptide which comprises the amino acid sequence represented by SEQ ID NO:2, by the 1st to 166th positions of the amino acid sequence represented by SEQ ID NO:2, or by SEQ ID NO:4

are useful as the active ingredient of a medicinal composition for suppressing expression of type I collagen, fibronectin and/or MCP-1 in fibrotic organs (particularly the kidney or the bladder). In addition, the polypeptides described in the aforementioned (a) to (c) are useful as the active ingredient of a medicinal composition for improving and/or preventing fibrosis of organs (particularly the kidney or the bladder).

According to the invention, the polypeptide of the invention can be administered to an animal, preferably a mammal (particularly human), alone or together with a pharmaceutically acceptable general carrier as occasion demands. Also included in the invention is a method for suppressing expression of type I collagen, fibronectin and/or MCP-1 (preferably a method for improving and/or preventing fibrosis), which comprises administering an effective amount of the polypeptide described in the aforementioned (a) to (c), a polynucleotide coding for said polypeptide or an expression vector containing said polynucleotide to an object which requires expression suppression of type I collagen, fibronectin and/or MCP-1 (preferably an object which requires improvement and/or prevention of fibrosis).

In addition, the polypeptide described in the aforementioned (a) to (c) can be used for producing a medicinal composition for suppressing expression of type I collagen, fibronectin and/or MCP-1, or a medicinal composition for improving and/or preventing fibrosis.

The medicinal composition for suppressing expression of type I collagen, fibronectin and/or MCP-1, or medicinal composition for improving and/or preventing fibrosis, according to the invention can be prepared using additive agents generally used for their formulation in response to the active ingredient.

The administration method is not particularly limited, with the proviso that it is administered in such a manner that the effective amount reaches the affected part. For example, oral administration by tablets, pills, capsules, granules, fine subtilaes, powders, oral solutions or the like, or parenteral administration can be exemplified. As the parenteral administration, for example, a systemic administration (e.g., intravenous administration, intraarterial administration, subcutaneous administration, intramuscular administration or the like), a topical administration (e.g., intravesical administration or the like), a transmucosal administration (e.g., intranasal administration, buccal administration or the like), an intestinal administration (e.g., suppository administration or the like) and the like can be exemplified, but in the case of fibrotic conditions of the bladder, intravesical administration can be particularly selected. [Goodman & Gilman's The Pharmacological Basis of Therapeutics, Tenth Edition, Hardman J.G., Limbird L.E. and Gilman A.G., The McGraw-Hill Companies, Inc., 2001]. In addition, when the aforementioned active ingredient is a polypeptide which undergoes influence of digestive enzyme, it is desirable to employ intravenous injection or the like parenteral administration, or to use a formulation technique which renders possible delivery of the aforementioned polypeptide without degradation to a lower part of the digestive organs where influence of the digestive enzyme is small (e.g., the jejunum, the ileum, the colon or the large intestine). AS such a formulation technique, for example, a sustained release preparation (e.g., International Publication pamphlet WO 94/06414), a colon release preparation (e.g., International Publication pamphlet WO 95/28963) or a timed release type or pulse release type preparation (e.g., International Publication pamphlet WO 93/05771) can be cited.

In the solid composition for oral administration, one or two or more active ingredients can be mixed with at least one pharmacologically acceptable inert diluent such as lactose, mannitol, microcrystalline cellulose, hydroxypropylcellulose, starch or the like, and it can contain a pharmacologically acceptable additive agent other than the aforementioned diluent, such as a lubricant, a disintegrating agent, a stabilizer, a solubilizing or solubilization assisting agent or the like. As occasion demands, tablets or pills can be coated with a sugar coat or a film of a gastric or enteric substance. The liquid composition for oral administration can contain a pharmacologically acceptable inert diluent (e. g., purified water or ethanol) and a pharmacologically acceptable additive agent such as emulsions, solutions, suspensions, syrups, elixirs, moistening agents, aromatics, or antiseptics.

As the injections for parenteral administration, a solubilizing agent, a preservative, a stabilizing agent, an emulsifying agent, a soothing agent, a tonicity agent, a buffer agent, a filler, a coloring agent, a thickener or the like additive agent can be formulated. As the aforementioned solubilizing agent, cyclodextrins and the like can be exemplified. As the aforementioned preservative, methyl p-benzoate and the like can be exemplified. As the aforementioned emulsifying agent, lecithin and the like can be exemplified. As the aforementioned soothing agent, benzyl alcohol and the like can be exemplified. As the aforementioned tonicity agent, sodium chloride and the like can be exemplified. As the aforementioned filler, maltose and the like can be exemplified. As the aforementioned thickener, hyaluronic acid and the like can be exemplified. The aforementioned injections can be sterilized, for example, by filtration through a bacteria retaining filter, formulation of a germicide or radiation irradiation. In addition, they can be produced as an aseptic solid composition and used by aseptically dissolving in sterile water or other aseptic medium for injection when used.

The dose can be optionally decided, for example, by taking symptoms, age or sex of each subject to be administered and the like into consideration. In the case of oral administration for example, the dose is generally approximately from 0.1 to 100 mg, preferably from 0.1 to 50 mg, per day per adult (as 60 kg in body weight). In the case of parenteral administration, it is from 0.01 to 50 mg, preferably from 0.01 to 10 mg, as a form of injections.

Regarding the gene delivery method for gene therapy, (1) a virus vector, (2) a non-virus vector or (3) naked DNA can be used. Also, regarding the method of gene therapy, a method in which a gene transferred cell is transplanted into each patient (ex vivo method) or a method in which the gene itself is injected directly into each individual without mediating cells *(in vivo* method) can be used [Edited by Y. Niitsu, Protein, Nucleic Acid, Enzyme (Supplement) "Gene Therapy", vol. 40, no. 17, 1995].

The administration method is not particularly limited, with the proviso that it is administered in such a manner that the effective amount reaches the affected part. For example, therapeutic methods by a systemic administration (e.g., intravenous administration, intraarterial administration, subcutaneous administration, intramuscular administration, oral administration or the like), a topical administration (e.g., intravesical administration or the like), a transmucosal administration (e.g., intranasal administration, endotracheal administration, buccal administration or the like), an intestinal administration (e.g., suppository administration or the like) and the like are included, but in the case of fibrotic conditions of the bladder, intravesical administration is desirable [Goodman & Gilman's The Pharmacological Basis of Therapeutics, Tenth Edition, Hardman J.G., Limbird L.E. and Gilman A.G., The McGraw-Hill Companies, Inc., 2001].

In addition, a medicinal composition for suppressing expression of type I collagen, fibronectin and/or MCP-1, or a medicinal composition for improving and/or preventing fibrosis, can be prepared by mixing the polynucleotide coding for the polypeptide of the invention or the expression vector of the invention with a pharmacologically acceptable carrier or solvent (e.g., physiological saline, pH buffer solution, stabilizing agent, preservative, suspending agent or the like). Also, for example, a technique for embedding the vector itself in a biodegradable gel for the purpose of improving effect of the gene therapy. Illustratively, a method in which release of DNA is sustained using attelo collagen (KOKEN Tokyo, Japan) (T. OCHIYA *et al., Nature Medicine,* 5, 707 - 710, 1999) can be used. Alternatively, phospholipid and/or cholesterol can be formulated for the purpose of improving cell transfer efficiency of the gene.

<Screening tool of the invention and its use for screening>

Also included in the invention is (1) a tool for screening a fibrosis improving agent, comprising (i) the promoter type polynucleotide of the invention, (ii) a polynucleotide which comprises a nucleotide sequence represented by the 1124th to 1525th positions of the nucleotide sequence represented by SEQ ID NO:23 wherein from 1 to 10 bases of the nucleotide sequence are deleted, substituted and/or inserted, and which has the promoter activity of the polynucleotide coding for the polypeptide of the invention, (iii) an expression vector which comprises the polynucleotide described in (i) or (ii), or (iv) a cell transfected with the aforementioned vector, and (2) use of (i) the promoter type polynucleotide of the invention, (ii) a polynucleotide which comprises a nucleotide sequence represented by the 1124th to 1525th positions of the nucleotide sequence represented by SEQ ID NO:23 wherein from 1 to 10 bases of the nucleotide sequence are deleted, substituted and/or inserted and which has the promoter activity of the polynucleotide coding for the polypeptide of the invention, (iii) an expression vector which comprises the polynucleotide described in (i) or (ii), or (iv) a cell transfected with the aforementioned vector, for screening a fibrosis improving agent.

According to this Description, the "screening tool" means a substance to be used in the screening (illustratively, a promoter type polynucleotide to be used for the screening, an expression vector comprising said polynucleotide or a cell expressing the promoter type polynucleotide). The "tool for screening a fibrosis improving agent" is a cell or promoter type polynucleotide to be contacted with a test compound in the screening method of the invention for screening a fibrosis improving agent (e.g., an agent for improving renal insufficiency or fibrotic conditions of the bladder). Use of the promoter type polynucleotide of the invention, the expression vector comprising said polynucleotide or the cell, for the screening of a fibrosis improving agent is also included in the invention.

<Screening method of the invention>

It was revealed that, when FREP as one of the polypeptides of the invention is over-expressed, expression of fibronectin and type I collagen from uriniferous tubule epithelial cells and their expression acceleration by TGF-β are suppressed (Example 6), acceleration of the expression MCP-1 from uriniferous tubule epithelial cells by BSA is suppressed (Example 7), and expression of fibronectin and type I collagen is suppressed in the presence of FREP-N as one of the polypeptide of the invention. It is known that overproduction of extracellular matrixes such as fibronectin and type I collagen becomes the cause of fibrotic conditions, and MCP-1 is a factor relating to the advance of glomerulonephritis. Based on these facts, it was found that the aforementioned substance which accelerates expression of the polypeptide of the invention is useful as an agent for improving and/or preventing fibrotic conditions. Thus, it was found that an agent for improving and/or preventing fibrotic conditions can be screened using the promoter activity of the promoter type polynucleotide of the invention as the index.

That is, a method for screening a substance having the action to improve fibrotic conditions, which uses a polynucleotide as the screening tool of the invention (namely, the promoter type polynucleotide of the invention, or a polynucleotide which comprises a nucleotide sequence represented by the 1124th to 1525th positions of the nucleotide sequence represented by SEQ ID NO:23 wherein from 1 to 10 bases of the nucleotide sequence are deleted, substituted and/or inserted, and which has the promoter activity of the polynucleotide coding for the polypeptide of the invention) and uses change in said promoter activity as the index, is included in the screening method of the invention.

More illustratively, the following method is included in the screening method of the invention.

A method for screening a fibrosis improving agent, characterized in that it comprises
a step for allowing a cell expressing <u>a polynucleotide as the screening tool of the invention</u> to contact with a substance to be tested,
a step for detecting the promoter activity, and
a step for selecting a substance which accelerates the promoter activity.

As an embodiment of the screening method of the invention, a reporter gene assay system can be cited. The reporter gene assay (Tamura *et al.,* Tensha Inshi Kenkyu-ho (Method for Studying Transcription Factors), Yodo-sha, 1993) is a method for detecting expression regulation of a gene using expression of a reporter gene as the marker. In general, expression regulation of a gene is controlled by a moiety called promoter region existing in its 5' upstream side, and the gene expression quantity at the stage of transcription can be estimated by measuring the activity of this promoter. When a test substance activates the promoter, it activates transcription of a reporter gene arranged in the downstream of the promoter region. Thus, the promoter activation action, namely the expression acceleration action, can be detected by replacing it by the expression of the reporter gene. Accordingly, the action of a test substance upon expression regulation of the polypeptide of the invention can be detected by replacing it by the expression of a reporter gene, by a reporter gene assay which uses a polynucleotide as the screening tool of the invention. The "reporter gene" fused with a polynucleotide as the screening tool of the invention (e.g., a sequence consisting of the nucleotide sequence represented by SEQ ID NO:23) is not particularly limited with the proviso that it is generally used, but a enzyme gene or the like whose quantitative measurement can be easily carried out is desirable. For example, a bacterial transposon derived chloramphenicol acetyltransferase gene (CAT), a firefly derived luciferase gene (Luc), a jellyfish derived green fluorescent protein gene (GFP) and the like can be cited. The reporter gene should be fused functionally with a polynucleotide as the screening tool of the invention. By comparing expression quantities of a reporter gene in which a test substance is contacted with a cell transfected by the reporter gene fused with the promoter of the invention and in which they are not contacted, changes in the test substance-dependent transcription induction activity can be analyzed. By carrying out the aforementioned steps, screening of a fibrosis improving agent can be carried out. Illustratively, the aforementioned screening can be carried out by the method described in Example 8.

Though the test substance to be used in the screening method of the invention is not particularly limited, its examples include commercially available compounds (including peptides), various conventionally known compounds registered in the chemical file (including peptides), a group of compounds obtained by the combinatorial chemistry technique (N. Terrett *et al., Drug Discov. Today,* 4(1): 41, 1999), culture supernatants of microorganisms, natural components derived from plants and marine organisms, animal tissue extracts, or compounds (including peptides) prepared by chemically or biologically modifying compounds (including peptides) selected by the screening method of the invention.

<Method for inspecting chronic renal insufficiency and/or fibrotic conditions of the bladder>

Expression quantity of the polynucleotide coding for the polypeptide of the invention can be examined by using a probe which hybridizes with the polynucleotide of the invention under a stringent condition, and detection and diagnosis of chronic renal insufficiency and/or fibrotic conditions of the bladder can be carried out using decrease in the expression quantity (preferably expression quantity in the kidney or bladder) as the index. The term "stringent condition" as used herein means a condition under which nonspecific binding does not occur, and it illustratively means a condition in which a 0.1 x SSC (saline-sodium citrate buffer) solution containing 0.1 % sodium dodecyl sulfate (SDS) is used , and the temperature is 65°C. A DNA fragment of at least 15 bp in chain length having a polynucleotide of the invention (or complementary sequence thereof) or a part of the polynucleotide of the invention is useful as the aforementioned probe.

More illustratively, by the detection method of chronic renal insufficiency and/or fibrotic conditions of the bladder, whether or not it is chronic renal insufficiency and/or fibrotic conditions of the bladder can be detected by analyzing a bonded body of a polynucleotide coding for the polypeptide of the invention (e.g., mRNA or cDNA derived therefrom) and the aforementioned probe, by allowing the aforementioned probe to contact with a test sample. When amount of the aforementioned bonded body, namely amount of the polynucleotide coding for the polypeptide of the invention, is decreased in comparison with the case of normal parson, the result can be judged (namely diagnosed) as chronic renal insufficiency and/or fibrotic conditions of the bladder. Thus, the polynucleotide of the invention is useful for the detection and diagnosis of chronic renal insufficiency and/or fibrotic conditions of the bladder.

As the detection method of chronic renal insufficiency and/or fibrotic conditions of the bladder, a method in which the expression level is measured by detecting the polypeptide of the invention is possible, in addition to the method in which the expression level of the polynucleotide of the invention is measured as described in the above. As such an inspection method, for example, western blotting, immunoprecipitation, ELISA or the like method can be used making use of an antibody which binds to the polypeptide of the invention in a test sample, preferably an antibody which binds specifically to the polypeptide of the invention. The polypeptide of the invention can be used as the standard amount in determining amount of the polypeptide of the invention contained in a test sample. In addition, the polypeptide of the invention is useful for preparing an antibody which binds to the polypeptide of the invention. When amount of the polypeptide of the invention is decreased in comparison with the case of a normal person, the result can be judged as chronic renal insufficiency and/or fibrotic conditions of the bladder.

<Production method of medicinal composition for improving fibrotic conditions>

Also included in the invention is a method for producing a medicinal composition for improving fibrotic conditions, characterized in that it comprises a step for carrying out screening using the screening method of the invention, and a step for preparing pharmaceutical preparations.

A pharmaceutical preparation containing a substance obtained by the screening method of the invention as the active ingredient can be prepared by using a carrier, a filler and/or other additive agents generally used in the formulation.

Regarding the oral administration, solid composition and liquid composition for oral administration, parenteral administration, injections for parenteral administration and dose, they can be carried out in the same manner as in the <Medicinal composition of the invention for improving and/or preventing fibrosis> of this Description.

Examples

The following describes the invention in detail based on examples, but the invention is not restricted by said examples. In this connection, unless otherwise noted, these can be carried out in accordance with conventionally known methods ("Molecular Cloning" Sambrook, J *et al,* Cold spring Harbor Laboratory Press, 1989, "Tanpaku Jikken Protocol (Protein Experimentation Protocol)" Shuhjun-sha, 1997, and the like). In addition, when commercially available reagents and kits are used, these can be carried out in accordance with the instructions of the commercial products.

<Example 1> Cloning of human kidney derived gene and construction of expression vector

(1) Cloning of complete length cDNA of human kidney derived gene and preparation of expression vector
Primers of the nucleotide sequences represented by SEQ ID NO:5 and SEQ ID NO:6 were synthesized (Proligo), and amplification of complete length cDNA from a human kidney derived cDNA library (Clontech) by PCR was attempted using said primers. The PCR reaction was carried out using a DNA polymerase (TAKARA LA Taq;

Takara Shuzo) and repeating, after 95°C (5 minutes), a cycle of 95°C (30 seconds), 55°C (30 seconds) and 72°C (2 minutes) 37 times. The primer shown by SEQ ID NO:6 was designed in such a manner that a vector derived V5 epitope (derived from the V protein of paramyxovirus SV5, Southern JA (1991) *J. Gen. Virol.,* 72, 1551 - 1557, 1991) and 6 x His tag (Lindner P (1997) BioTechniques 22, 140 - 149) are added to the 3' side after cloning. The PCR product was separated by an agarose gel electrophoresis to confirm that a DNA fragment of about 2000 base pairs was amplified, and then this DNA fragment in the reaction liquid was cloned into an expression vector (pcDNA3.1/V5-His-TOPO; Invitrogen) using TOPO TA Cloning System (Invitrogen). Nucleotide sequence of the inserted DNA fragment in the thus obtained plasmid (named pcDNA-hFREP) was determined using a sequencing kit (Applied Biosystems) and a sequencer (ABI 3700 DNA Sequencer, Applied Biosystems). As a result, it was confirmed that this is a clone containing the nucleotide sequence represented by SEQ ID NO: 1. Based on this, open reading frame of the gene represented by SEQ ID NO:1 was established. This gene was named FREP gene.
(2) Cloning of complete length cDNA of rat FREP gene

Primers of the nucleotide sequences represented by SEQ ID NO:7 and SEQ ID NO:8 were synthesized (Proligo), and amplification of complete length cDNA from a rat kidney derived cDNA library (Clontech) by PCR was attempted using said primers. The PCR reaction was carried out using a DNA polymerase (TAKARA LA Taq; Takara Shuzo) and repeating, after 95°C (5 minutes), a cycle of 95°C (30 seconds), 55°C (30 seconds) and 72°C (2 minutes) 37 times. The PCR product was separated by an agarose gel electrophoresis to confirm that a DNA fragment of about 2000 base pairs was amplified, and then this DNA fragment in the reaction liquid was cloned into an expression vector (pcDNA3.1/V5-His-TOPO; Invitrogen) using TOPO TA Cloning System (Invitrogen). Nucleotide sequence of the inserted DNA fragment in the thus obtained plasmid was determined using a sequencing kit (Applied Biosystems) and a sequencer (ABI 3700 DNA Sequencer, Applied Biosystems). As a result, it was confirmed that this is a clone containing the nucleotide sequence represented by SEQ ID NO:3. Based on this, open reading frame of the gene represented by SEQ ID NO:3 was established. Since the nucleotide sequence of this gene showed the most high homology with the human FREP gene, showing about 80% of homology in comparison with the nucleotide sequence of human FREP gene, it was revealed that this gene is a rat orthologue of the human FREP gene. Since homology of rat FREP with human FREP at the deduced amino acid level was about 80%, showing high homology, it was considered that the rat FREP is a protein having the function equivalent to the human FREP. In this connection, the aforementioned "homology" means a value obtained using parameters prepared with default by Clustal program (Higgins and Sharp, *Gene,* 73, 237 - 244, 1998; Thompson et al., *Nucl. Acids Res.,* 22, 4673 - 4680, 1994) retrieval. The aforementioned parameters are as follows.

As Pairwise Alignment Parameters
K tuple 1
Gap Penalty 3
Window 5
Diagonals Saved 5

<Example 2> Preparation of transformed cell expressing FREP protein

(1) Preparation of FREP expression cell

i) Transient expression of human FREP protein in COS-1 cell

The aforementioned expression plasmid pcDNA-hFREP prepared in Example 1(1) was introduced into COS-1 cell. COS-1 cell was cultured until it became confluent state, by adding 10 ml of a minimum essential medium DMEM (Gibco) containing 10% fetal bovine serum (Sigma) to a culture dish (10 cm in diameter, Asahi Techno Glass). Using a lipofection reagent (lipofectoamine 2000; Invitrogen) and in accordance with the protocol attached to the lipofection reagent, this cell was transiently transfected with pcDNA3.1 (empty vector) or pcDNA-hFREP (3 μg). After 24 hours of culturing, the medium was removed, the cells were washed with a phosphate buffer liquid (to be referred to as PBS hereinafter), and then the cells were lysed by adding 0.25 ml of a cell lysis liquid (50 mM Tris-HCl buffer (pH 8.0), 150 mM sodium chloride, 1% NP-40, 2 mM phenylmethylsulfonyl fluoride (PMSF)). The cell lysate was prepared in the same manner also for the un-introduced COS-1 cell.

ii) Stable expression of human FREP protein NRK52E cell

The expression plasmid pcDNA-hFREP was introduced into NRK52E cell which is a cell strain derived from rat uriniferous tubule epithelial cell. The NRK52E cell was cultured until it became confluent state, by adding 10 ml of a minimum essential medium DMEM (Gibco) containing 1% fetal bovine serum (JRH) to a culture dish (10 cm in diameter, Asahi Techno Glass). Using the lipofection reagent (lipofectoamine 2000; Invitrogen) and in accordance with the protocol attached to the lipofection reagent, this cell was transfected with pcDNA3.1 (empty vector) or pcDNA-hFREP (3 μg). After 24 hours of culturing, the medium was exchanged with fresh one, and further thereafter, 0.5 mg/ml in final concentration of G418 (Nakalai Tesque) was added to the medium to carry out subculture, and cells seemingly acquired the drug resistance through the integration of the expression plasmid into chromosome were selected.

(2) Detection of FREP protein

A 20 μl portion of the aforementioned lysate of empty vector expression cell, human FREP expression cell or un-introduced COS-1 cell of <Example 2>(1)i) was mixed with 20 μl of a 2 x concentration SDS sample buffer (125 mM Tris-HCl (pH 6.8), 3% sodium lauryl sulfate, 20% glycerol, 0.14 M β-mercaptoethanol, 0.02% Bromophenol Blue) and treated at 100°C for 5 minutes, and then proteins contained in the sample were separated by carrying out 10% SDS polyacrylamide gel electrophoresis. Proteins in the polyacrylamide gel were transferred onto a polyvinylidene difluoride (PVDF, Nippon Millipore) membrane using a semidry type blotting device (Bio-Rad), and then detection of the FREP protein on said PVDF membrane was carried out by western blotting in accordance with the general method. A monoclonal antibody capable of recognizing V5 epitope fused to the C-terminal of FREP (Invitrogen) was used as the primary antibody, and anti-mouse IgG-HRP fused antibody (Amersham Bioscience) was used as the secondary antibody. It was confirmed that a protein of about 80 kDa which indicates a FREP-V5-HIS6 fusion protein consisting of 680 amino acids containing a C-terminal side tag consisting of 45 amino acids is detected depending on the presence of the expression vector pcDNA-hFREP (Fig. 1). Based on this, it was revealed that the aforementioned FREP gene cloned in the cultured cell is surely expressed for its complete length region and forms stable structure as the protein.

After washing the aforementioned empty vector-introduced and FREP-expressed NRK52E cells of <Example 2>(1)ii) with PBS, the cells were lysed by adding 0.25 ml of the aforementioned cell lysis liquid, and SDS polyacrylamide gel electrophoresis and western blotting were carried out in the same manner as the case of the aforementioned COS-1 cell. As a result, it was confirmed that a protein of about 80 kDa which indicates the FREP-V5-HIS6 fusion protein consisting of 680 amino acids containing the C-terminal side tag consisting of 45 amino acids is detected depending on the presence of the expression vector pcDNA-hFREP. Based on this, it was revealed that the FREP gene is surely expressed in the isolated transformed NRK52E cell.

<Example 3> Expression distribution analysis of human FREP gene in respective tissues

Expression distribution of the human FREP gene of the invention was analyzed by RT-PCR. Poly A+ RNA (5 mg) (Clontech) derived from corresponding human organ was allowed to undergo the reaction at 37°C for 30 minutes by adding a DNase (Promega). Using total amount of this DNase-treated poly A+ RNA, cDNA was synthesized using SUPERSCRIPT First-Strand Synthesis System for RT-PCR (Invitrogen) and in accordance with the protocol attached to the kit. The synthesized cDNA was dissolved in 900 μl of sterilized water. Using a pair of primers represented by SEQ ID NO:9 and SEQ ID NO:10, an attempt was made to amplify a partial cDNA fragment of the human FREP gene represented by SEQ ID NO:1 from the aforementioned cDNA derived from a corresponding tissue by PCR, and the presence or absence of FREP in respective tissues was examined. Using a DNA polymerase (TAKARA LA Taq; Takara Shuzo), 1 μl portion of each solution of the cDNA synthesized in the aforementioned manner was heated at 95°C (5 minutes) and then a PCR cycle of 95°C (30 seconds), 55°C (30 seconds) and 72°C (30 seconds) was repeated 40 times. When each of the thus obtained PCR products was separated by an agarose gel electrophoresis, a DNA fragment of about 200 base pairs considered to be containing a partial fragment for the desired human FREP partial fragment was amplified from each of the cDNA samples derived from the kidney, the bladder and the prostate. Based on this, it was revealed that expression of the human FREP gene represented by SEQ ID NO:1 is expressed in an organ capable of inducing fibrosis.

<Example 4> Measurement of FREP expression quantities in normal and chronic renal insufficiency model rats

Based on the aforementioned knowledge, it was found that the human FREP protein of the invention is expressed in the kidney and the like organs where organ fibrosis can be seen, so that it was predicted that the polypeptide of the invention is concerned in chronic renal insufficiency. Accordingly, the expression quantity of messenger RNA (mRNA) of rat FREP gene in the kidney of a chronic renal insufficiency model rat, 5/6 kidney extraction rat (Shea *et al., Am. J. Pathol.:* 100, 513 - 528, 1980), was measured and compared.

Regarding the gene expression quantity, expression quantity of rat FREP gene was measured, and corrected based on the simultaneously measured expression quantity of glyceraldehyde 3-phosphate dehydrogenase (G3PDH) gene. As the measuring system, PRISM™ 7900 Sequence Detection System and SYBR Green PCR Master Mix (Applied Biosystems) were used. In this measuring system, expression quantity of the gene of interest is determined by the real time detection and determination of the fluorescence of SYBR Green I pigment incorporated by the double-stranded DNA amplified by PCR.

Illustratively, it was measured by the following procedure.

(1) Preparation of total RNA

Animals after 2, 4 and 6 weeks of the 5/6 kidney extraction operation carried out using 5 male Wistar rats of 8 weeks of age (Japan S L C) as the chronic renal insufficiency model rats, and animals after 2 weeks of fake operation carried out using 5 male Wistar rats of 8 weeks of age (Japan S L C) as normal control were prepared. The 5/6 kidney extraction operation and fake operation were carried out in accordance with the method of Shea *et al.* (Shea *et al., Am. J Pathol.:* 100, 513 - 528, 1980). Total RNA was prepared from the kidney of each of the aforementioned rats using a reagent for RNA extraction (Isogen; Nippon Gene) in accordance with the instructions thereof. The thus prepared total RNA was dissolved in sterile water and stored at - 80°C.

(2) Synthesis of single-stranded cDNA

Reverse transcription of total RNA to single-stranded cDNA was carried out in a system of 20 µl using 1 µg of RNA and using an enzyme for reverse transcription reaction (PowerScript™ reverse transcriptase; Clontech). After the reverse transcription, 180 µl of sterile water was added thereto and stored at -20°C.

(3) Preparation of PCR primers

The following 4 primers named FREP-F, FREP-R, G3PDH-F and G3PDH-R (SEQ ID NOs:11 to 14) were designed as the PCR primers described in the item (4). A combination of SEQ ID NO:11 and SEQ ID NO:12 was used for the FREP gene, and a combination of SEQ ID NO:13 and SEQ ID NO:14 for the G3PDH gene.

(4) Measurement of gene expression quantity

Real time measurement of PCR amplification by PRISM™ 7900 Sequence Detection System was carried out in a system of 10 µl in accordance with the instructions. In each system, 4 µl of single-stranded cDNA, 5 µl of 2 x SYBR Green reagent and 3 pmol of each primer were used. In this connection, in preparing a calibration curve, 0.1 µg/µl of a rat genomic DNA (Clontech) was appropriately diluted, and a 4 µl portion thereof was used instead of the single-stranded cDNA. PCR was carried out by heating at 95°C for 10 minutes and then repeating 45 cycles of a process consisting of 2 steps of 95°C for 15 seconds and 59°C for 60 seconds.

Expressed amount of the rat FREP gene in each sample was corrected by the expressed amount of G3PDH gene based on the following formula.

[Expressed amount of FREP after correction] = [expressed amount of FREP gene (raw data)]/[expressed amount of G3PDH gene (raw data)]

As a result of the above, as shown in Fig. 2, it was revealed that expression of rat orthologue gene of the FREP of the invention is decreased in the kidney of the chronic renal insufficiency model rat. Accordingly, it is considered that the FREP of the invention induces chronic renal insufficiency due to functional reduction in the kidney.

As described in the foregoing, it was difficult to detect early stage renal function disorder by the conventional renal function evaluation methods typified by the GFR measurement. Also, it was expected to start treatment of the renal function reduction by finding it at more early stage. In this Example, since decrease in the expressed amount of FREP gene was observed in and after 2 weeks of the 5/6 kidney extraction operation, it was revealed that the expressed amount of FREP gene can be used as an early stage index of chronic renal insufficiency before the appearance of a standard clinical indicator.

<Example 5> Measurement of FREP expression quantity in normal and bladder fibrosis-induced rats

The expression quantity of messenger RNA (mRNA) of FREP gene in the bladder of a urethral stricture rat (Malmgren A. *et al., J. Urol.:* 142, 1134 - 1138, 1989) was measured and compared with that of a nonnal rat. It is known that fibrosis is induced in the bladder of this rat (Chaqour *et al., Am. J Physiol.:* 283, E 765 - E 774, 2002).

Regarding the gene expression quantity, expression quantity of the rat FREP gene of the invention was measured, and corrected based on the simultaneously measured expression quantity of G3PDH gene. As the measuring system, PRISM™ 7900 Sequence Detection System and SYBR Green PCR Master Mix (Applied Biosystems) were used. In this measuring system, expression quantity of the gene of interest is determined by the real time detection and determination of the fluorescence of SYBR Green I pigment incorporated by the double-stranded DNA amplified by PCR.

Illustratively, it was measured by the following procedure.

(1) Preparation of total RNA

Rats in which bladder fibrosis was induced were prepared by strangulating each urethra of 4 female SD (Sprague Dawley) rats of 10 weeks of age (Charles River Japan) to 1 mm in diameter with s silk thread and rearing the animals for 6 weeks. Four female SD (Sprague Dawley) rats of 10 weeks of age (Charles River Japan) reared for 6 weeks after subjecting to false operation were prepared as a normal control. The preparation method of rat in which bladder fibrosis was induced and the method of false operation were carried out in accordance with the methods of Malmgren A. *et al.* The bladder of each of the aforementioned rat in which bladder fibrosis was induced and normal rat was extracted, and total RNA of the bladder was prepared using a reagent for RNA extraction (Isogen) in accordance with the instructions thereof and then subjected to a DNase treatment on RNeasy mini-column (Qiagen) in accordance with the instructions thereof. The thus prepared total RNA was dissolved in sterile water and stored at -80°C.

(2) Synthesis of single-stranded cDNA

Reverse transcription of total RNA to single-stranded cDNA was carried out in a system of 20 µl using 1 µg of RNA and using an enzyme for reverse transcription reaction (ThermoScript™ reverse transcriptase; Invitrogen). After the reverse transcription, 180 µl of sterile water was added thereto and stored at -20°C.

(3) Measurement of gene expression quantity

Real time measurement of PCR amplification by PRISM™ 7900 Sequence Detection System was carried out in a system of 10 µl in accordance with the instructions thereof in the same manner as in the aforementioned Example 4, and expressed amount of the rat FREP gene in each sample was corrected by the expressed amount of G3PDH gene based on the following formula.

[Expressed amount of FREP after correction] = [expressed amount of FREP gene (raw data)]/[expressed amount of G3PDH gene (raw data)]

As a result, it was revealed that the expressed amount of rat FREP gene of the invention is decreased in the rat in which bladder fibrosis was induced, by a factor of about 48% in comparison with the normal animal. Accordingly, it was found that reduction of expressed amount of the invention induces bladder fibrosis due to functional reduction in the bladder.

Interstitial cystitis is differentially diagnosed by cystoscopic findings and pain in the bladder region, but a clinical indicator for objectively supporting the diagnosis has not been found yet. The inventors have found that expressed amount of the FREP gene is decreased in the bladder of a rat in which bladder fibrosis was induced, in comparison with a normal animal, and revealed that the FREP gene is useful for the diagnosis of fibrotic conditions of the bladder.

<Example 6> Effect of FREP on fibronectin and type I collagen

It has been reported that TGF-β accelerates expression of fibronectin gene in NRK52E cell (Yokoi H. *et al., Am. J Phydiol. Renal Physiol.:* 282, F 933 - F 942, 2002). Also, it has been reported that expression quantity of type I collagen is accelerated by TGF-β treatment in the case of NRK52E cell (Creely J.J. *et al., Am. J Pathol.:* 140, 45 - 55, 1992). Accordingly, change in the fibronectin gene expression quantity when TGF-β was not added or added to a cultured medium of the aforementioned rat FREP stable expression cell was measured, and effect of FREP on the fibronectin expression acceleration induced by fibronectin expression or TGF-β addition was observed. In addition, change in the expression quantity of type I collagen gene as one of the extracellular matrix constituting components was also measured, and effect of FREP was observed. Regarding the gene expression quantity (mRNA expression quantity), expression quantities of rat fibronectin and type I collagen were measured, and corrected based on the simultaneously measured expression quantity of G3PDH gene. As the measuring system, PRISM™ 7900 Sequence Detection System and SYBR Green PCR Master Mix (Applied Biosystems) were used. Illustratively, they were measured by the following procedure.

(1) Preparation of total RNA

The FREP expressing NRK52E cell or NRK52E cell of <Example 2>(1)ii) was inoculated into a collagen-coated dish (6 well dish, Asahi Techno Glass) in 1 x $10^5$ cells/well potions and cultured overnight, and then TGF-β (Sigma) was not added or added thereto. After 24 hours, the cells were recovered, and total RNA was prepared using RNeasy mini-column (Qiagen) in accordance with the instructions thereof. In this case, DNase treatment was simultaneously carried out in accordance with the same instructions. The thus prepared total RNA was dissolved in sterile water and stored at -80°C.

(2) Synthesis of single-stranded cDNA

Reverse transcription of total RNA to single-stranded cDNA was carried out in a system of 20 µl using 0.5 µg of RNA and using an enzyme for reverse transcription reaction (ThermoScript™ reverse transcriptase; Invitrogen). After the reverse transcription, 180 µl of sterile water was added thereto and stored at -20°C.

(3) Preparation of PCR primers

The following 4 oligonucleotides named FN-F, FN-R, Col-F and Col-R (SEQ ID NOs:15 to 18) were designed as the PCR primers described in the item (4). A combination of SEQ ID NO: 15 and SEQ ID NO: 16 was used for the fibronectin gene, a combination of SEQ ID NO: 17 and SEQ ID NO:18 for the type I collagen gene, and the combination of SEQ ID NO: 13 and SEQ ID NO:14 prepared in <Example 4>(3) for the G3PDH gene.

(4) Measurement of gene expression quantity

Real time measurement of PCR amplification by PRISM™ 7900 Sequence Detection System was carried out in a system of 10 µl in accordance with the instructions, and expressed amounts of the rat fibronectin gene and rat type I collagen gene in each sample were corrected by the expressed amount of G3PDH gene based on the following formula.

[Expressed amount of fibronectin after correction] = [expressed amount of fibronectin gene (raw data)]/[expressed amount of G3PDH gene (raw data)]

[Expressed amount of type I collagen after correction] = [expressed amount of type I collagen gene (raw data)]/[expressed amount of G3PDH gene (raw data)]

As a result, it was found that expressed amounts of fibronectin and type I collagen at the time of TGF-β un-addition or addition are decreased in comparison with the control cell, in the cell which stably over-expresses the FREP of the invention. It was found that, in the cell which stably over-expresses the FREP of the invention, the expressed amount of fibronectin is decreased by a factor of about 64% at the maximum (at the time of the addition of 3 ng/ml of TGF-β) in comparison with the control cell (Fig. 3). It was found also that the expressed amount of type I collagen is decreased by a factor of about 94% at the maximum (at the time of the addition of 1 ng/ml of TGF-β) (Fig. 4). Accordingly, it was found that the FREP of the invention suppresses expression of fibronectin and type I collagen in the uriniferous tubule epithelial cell and their expression acceleration by TGF-β and decreases excess extracellular matrix production in the uriniferous tubule as a result, and thereby improves and/or prevents fibrosis of the uriniferous tubule interstitium and further delays and prevents advance of functionally incomplete morbid states of the kidney derived therefrom.

<Example 7> Effect of FREP on the MCP-1 expression acceleration induced by the addition of BSA

It is already reported that expression of MCP-1 is increased when bovine serum albumin (BSA) is added to a culture medium of rat primary culture proximal uriniferous tubule epithelial cell (Wang Y. *et al., J. Am. Soc. Nephrol.:* 8, 1537 - 1545, 1997). Accordingly, change in the MCP-1 expression quantity when BSA is added to a culture medium of the aforementioned FREP stable expression cell was measured, and effect of FREP on the MCP-1 expression acceleration induced by the addition of BSA was observed. Regarding the gene expression quantity, expression quantity of rat MCP-1 gene was measured and corrected based on the simultaneously measured expression quantity of G3PDH gene. As the measuring system, PRISM™ 7900 Sequence Detection System and SYBR Green PCR Master Mix (Applied Biosystems) were used.

Illustratively, it was measured by the following procedure.

(1) Preparation of total RNA

This was carried out in the same manner as in <Example 6>(1). However, BSA (Sigma) dissolved in PBS was added instead of TGF-β

(2) Synthesis of single-stranded cDNA

This was carried out in the same manner as in <Example 6>(2).

(3) Preparation of PCR primers

Oligonucleotide named MCP-F and MCP-R were designed as the PCR primers described in the item (4). A combination of SEQ ID NO:19 and SEQ ID NO:20 was used for the MCP-1 gene, and a combination of SEQ ID NO:13 and SEQ ID NO:14 prepared in <Example 4>(3) for the G3PDH gene.

(4) Measurement of gene expression quantity

Real time measurement of PCR amplification by PRISM™ 7900 Sequence Detection System was carried out in a system of 10 μl in accordance with the instructions in the same manner as in the aforementioned Example 6, and expressed amount of the rat MCP-1 gene in each sample was corrected by the expressed amount of G3PDH gene based on the following formula.

[Expressed amount of MCP-1 after correction] = [expressed amount of MCP-1 gene (raw data)]/[expressed amount of G3PDH gene (raw data)]

As a result, it was found that, in the cell which stably over-expresses the FREP of the invention, the expressed amount of MCP-1 is decreased by a factor of about 86% at the maximum in comparison with the control cell (Fig. 5). Accordingly, it is considered that the FREP of the invention suppresses expression acceleration of MCP-1 in the uriniferous tubule epithelial cell by proteinuria and which suppresses excess inflammatory reaction in the vicinity of uriniferous tubule as a result, and thereby prevents and delays advance of glomerulonephritis and further functionally incomplete morbid states of the kidney derived therefrom.

The inventors have found that the MCP-1 expression quantity is suppressed by the FREP over expression, and thereby revealed that the FREP gene is useful for the improvement of inflammatory disease of the kidney.

<Example 8> Detection of promoter activity of human FREP gene

(1) Isolation of promoter region of human FREP gene and preparation of reporter vector

A DNA fragment containing an upstream sequence of the human FREP gene, represented by SEQ ID NO: 23, was obtained by PCR (using a DNA polymerase (LA Taq DNA polymerase; Takara Shuzo), heating at 98°C (5 minutes), subsequently repeating 35 times of a cycle of 96°C (30 seconds), 55°C (30 seconds) and 72°C (90 seconds), and then heating at 72°C for 7 minutes) using the primers of SEQ ID NO:21 and SEQ ID NO:22 and using a human genomic DNA (Clontech) as the template. This DNA fragment was treated with restriction enzymes (*Bgl*II and *Hin*dIII; Takara Shuzo) and connected to a luciferase reporter vector (pGL3-Basic vector; Promega) which had been restriction enzyme-treated in the same manner, thereby constructing a reporter vector (pGL3-FR-1525bp). Thereafter, this pGL3-FR-1525bp was treated with restriction enzymes (*Hin*dIII and *Xba*I*;* Takara Shuzo), and the thus obtained DNA fragment (from the 1124th to 1525th positions of SEQ ID NO:23) was linked to the pGL3-Basic vector which had been treated with restriction enzymes (*Hin*dIII and *Nhe*I*;* Takara Shuzo), thereby constructing a reporter vector (pGL3-FR-402bp).

(2) Detection of promoter activity of human FREP gene

Each of the pGL3-FR-402bp and pGL3-FR-1525bp constructed in Example 8(1) or the pGL3-Basic as a negative control (100 ng/well) was transiently cotransfected in the 293 cell together with a β-galactosidase expression vector (pCMV-β-galactosidase control vector; Roche Diagnostics) (10 ng/well). The co-transfection was carried out by the same method of Example 2(1). After 24 hours of culturing, the medium was removed, the cells were washed with a phosphate buffer liquid (PBS). The cells were lysed by adding 80 μl per well of a cell lysis liquid (100 mM potassium phosphate (pH 7.8), 0.2% Triton X-100). A 100 μl potion of a luciferase substrate solution (Wako Pure Chemical Industries) was added to 20 μl of this cell lysate , and the luminescence was measured using a chemiluminescence measuring device (ML 3000 type, Dynatech Laboratories). In addition, β-galactosidase activity of the aforementioned cell lysate was separately measured and numerically expressed using a β-galactosidase activity detection kit (Galacto-Light Plus™ system; Applied Biosystems). Using this as the transfection efficiency of transgene, the aforementioned luciferase activity in each well was corrected.

As a result of the aforementioned test, the promoter activity of the FREP was detected by a factor of about 17 times of the negative control pGL3-Basic when pGL3-FR-402bp was used, and by a factor of about 9 times of the pGL3-Basic when pGL3-FR-1525bp was used (Fig. 6). Based on these results, it was found that an FREP promoter assay system can be constructed by the use of the polynucleotides represented by SEQ ID NO:23 and the 1124th to 1525th positions of SEQ ID NO:23, and a substance which accelerates FREP expression quantity can be screened by this assay system.

In addition, since the activity obtained when pGL3-FR-1525bp was used was about half in comparison with the case of using pGL3-FR-402bp, it was revealed that the region of from -1525 bp to -402 bp is concerned in the transcription suppression regulation. Since a compound capable of releasing this suppression regulation is expected to have the action to accelerate transcription of the FREP gene as a result, it became possible to screen such a substance by a screening method which uses pGL3-FR-1525bp.

<Example 9> Preparation of transformed cell which expresses human FREP-N

It is known that the N-terminal sequence of membrane-bound type or secretion type protein contains a sequence called signal peptide, typically having a length of from 16 to 30 residues and containing from 4 to 12 hydrophobic residues (Lawn Biochemistry, Igaku Shoin, 1991). Since such a signal peptide sequence is recognized also in the N-tenninal sequence of the FREP protein, it was assumed that the FREP protein is a membrane-bound type or secretion type protein in which its N-terminal part is exposed to the extracellular moiety.

(1) Preparation of expression vector which expresses human FREP-N
Primers of the nucleotide sequences represented by SEQ ID NO:24 and SEQ ID NO:25 were synthesized (Proligo), and amplification ofFREP-NcDNA by PCR was attempted using said primers and using the pcDNA-hFREP constructed in the aforementioned Example 1 as the template. The PCR reaction was carried out using a DNA polymerase (TAKARA LA Taq; Takara Shuzo) and repeating, after 94°C (7 minutes), a cycle of 94°C (30 seconds), 55°C (30 seconds) and 72°C (30 seconds) 25 times. The primer shown by SEQ ID NO:25 was designed in such a manner that a vector derived V5 epitope (derived from the V protein of paramyxovirus SV5, Southern J A (1991) *J Gen. Virol., 72,* 1551 - 1557, 1991) and 6 x His tag (Lindner P (1997) *BioTechniques* 22, 140 - 149) came in succession with the same frame of the triplet of FREP gene, in the 3' side after cloning. The PCR product was separated by an agarose gel electrophoresis to confirm that a DNA fragment of about 500 base pairs was amplified, and then this DNA fragment in the reaction liquid was cloned into an expression vector (pcDNA3.1/V5-His-TOPO; Invitrogen) using TOPO TA Cloning System (Invitrogen). Nucleotide sequence of the inserted DNA fragment in the thus obtained plasmid (named pcDNA-hFREP-N) was determined using a sequencing kit (Applied Biosystems) and a sequencer (ABI 3700 DNA Sequencer, Applied Biosystems). As a result, it was confirmed that this is a clone containing the 1st to 498th positions of the nucleotide sequence represented by SEQ ID NO:1.
(2) Transient expression of human FREP-N protein in COS-7 cell and purification thereof

The aforementioned expression plasmid pcDNA-hFREP-N prepared in Example 9(1) was introduced into COS-7 cell. COS-7 cell was cultured until it became confluent state, by adding 10 ml of a minimum essential medium DMEM (Gibco) containing 10% fetal bovine serum (Sigma) to a culture dish (10 cm in diameter, Asahi Techno Glass). Using a lipofection reagent (lipofectoamine 2000; Invitrogen) and in accordance with the protocol attached to the lipofection reagent, this cell was transiently transfected with pcDNA-hFREP-N (3 μg). After 24 hours of culturing, the medium was removed and exchanged with 5 ml of fresh minimum essential medium DMEM (Gibco) containing 10% fetal bovine serum (Sigma), and the culturing was further continued for 24 hours. Thereafter, the FREP-N protein was recovered from this culture medium using a commercially available nickel affinity column (HisTrap, Amersham Bioscience). After washing the column under an appropriate condition, this was eluted and fractionated with a buffer liquid containing high concentration imidazole. By treating each fraction by the same method of the aforementioned <Example 2>(2), the hFREP-N protein was separated and detected. As a result, the FREP-N protein was detected in an eluted fraction as a protein of about 26 kDa (Fig. 7). Based on this, it was revealed that the FREP-N protein is secreted into the extracellular moiety and present in a relatively stable form and can be purified as a uniform protein.

<Example 10> Anti-fibrosis action of human FREP-N protein

(1) Preparation of culture liquid containing human FREP N-terminal partial protein
The pcDNA-hFREP-N was transferred into COS-7 cell by the same method of the aforementioned Example 9, and after overnight culturing, the medium was exchanged with a minimum essential medium DMEM (Gibco), and the culturing was further continued for 24 hours. This culture liquid is a culture liquid containing the FREP-N protein. Culture liquid of gene un-transferred COS-7 cell was used as the negative control.
(2) Measurement of expression quantity of extracellular matrix gene in fibroblast

A rat kidney fibroblast, NRK49F cell, was inoculated into a 6 well dish (Asahi Techno Glass) at a density of 2 $\times 10^5$ cells/well and cultured overnight, and then the medium was exchanged with the aforementioned culture liquid containing FREP-N protein or the culture liquid of gene un-transferred COS-7 cell. The cells were recovered 24 hours thereafter, and by the same methods as in the aforementioned Example 6, total RNA was prepared, single-strand cDNA was synthesized therefrom and the gene expression quantity was measured.

As a result, it was found that the expressed amounts of fibronectin and type I collagen in the NRK49F cell treated with the culture liquid containing the FREP-N protein of the invention were decreased by about 57% and about 50%, respectively, than those in the NRK49F cell treated with the culture liquid of gene un-transferred COS-7 cell. Accordingly, it was found that the FREP-N protein of the invention suppresses expression of fibronectin and type I collagen and reduces extracellular matrix production in the interstitium, and thereby delays and prevents fibrosis of uriniferous tubule interstitium and further advance of functionally incomplete morbid state of the kidney derived therefrom.

Industrial Applicability

The polypeptide of the invention and the polynucleotide coding for the polypeptide of the invention are useful in diagnosing chronic renal insufficiency and/or fibrotic conditions of the bladder. Also, the polypeptide of the invention and the polynucleotide coding for the polypeptide of the invention are useful as medicinal compositions for improving and/or preventing fibrosis. The polynucleotide having the promoter activity which regulates expression of the polypeptide of the invention is useful for the screening of a fibrosis improving agent. A substance selected by said screening is useful as a candidate of a fibrosis improving agent.

Sequence Listing Free Text

An explanation of "Artificial Sequence" is described in the numerical entry <223> of the following Sequence Listing. Illustratively, each of the nucleotide sequences represented by SEQ ID NOs:22, 24 and 25 of the Sequence Listing is an artificially synthesized primer sequence.

While the invention has been describe with reference to specific embodiments thereof, changes and modifications obvious to one skilled in the art are included in the scope of the invention.

## Claims

1. A polypeptide which comprises the amino acid sequence represented by SEQ ID NO:2 or SEQ ID NO:4 and which suppresses expression of type I collagen, fibronectin and/or MCP-1, or a polypeptide which comprises an amino acid sequence, represented by SEQ ID NO:2 or SEQ ID NO:4 wherein from 1 to 10 amino acids of the amino acid sequence are deleted, substituted and/or inserted and which suppresses expression of type I collagen, fibronectin and/or MCP-1. ,

2. A polypeptide which comprises the amino acid sequence represented by SEQ ID NO:2 or SEQ ID NO:4.

3. A polypeptide which comprises an amino acid sequence represented by the 1st to 166th positions of the amino acid sequence represented by SEQ ID NO:2, or a polypeptide which comprises an amino acid sequence represented by the 1 st to 166th positions of the amino acid sequence represented by SEQ ID NO:2 wherein from 1 to 10 amino acids of an amino acid sequence are deleted, substituted and/or inserted, and which suppresses expression of type I collagen, fibronectin and/or MCP-1.

4. A polynucleotide which encodes the polypeptide described in claim 1 to claim 3.

5. An expression vector which comprises the polynucleotide described in claim 4.

6. A cell transfected with the expression vector described in claim 5.

7. A medicinal composition for improving and/or preventing fibrosis, which contains a polypeptide described in any one of the following (a) to (c) as the active ingredient;

   (a) a polypeptide which comprises the amino acid sequence represented by SEQ ID NO:2, by the 1 st to 166th positions of the amino acid sequence represented by SEQ ID NO:2, or by SEQ ID NO:4, and which suppresses expression of type I collagen, fibronectin and/or MCP-1,
   (b) a polypeptide which comprises an amino acid sequence represented by SEQ ID NO;2, by the 1 st to 166th positions of the amino acid sequence represented by SEQ ID NO:2, or by SEQ ID NO:4, wherein from 1 to 10 amino acids of the amino acid sequence are deleted, substituted and/or inserted, and which suppresses expression of type I collagen, fibronectin and/or MCP-1, and
   (c) a polypeptide which comprises the amino acid sequence represented by SEQ ID NO:2, by the 1st to 166th positions of the amino acid sequence represented by SEQ ID NO:2, or by SEQ ID NO:4.

8. A medicinal composition for improving and/or preventing fibrosis, which contains a polynucleotide coding for the polypeptide described in claim 7 as the active ingredient.

9. A polynucleotide which comprises (1) the nucleotide sequence represented by SEQ ID NO:23, or (2) a nucleotide sequence represented by the 1124th to 1525th positions of the nucleotide sequence represented by SEQ ID NO:23.

10. An expression vector which comprises the polynucleotide described in claim 9.

11. A cell transfected with the expression vector described in claim 10.

**12.** A tool for screening a fibrosis improving agent, comprising (1) a polynucleotide which comprises the polynucleotide described in claim 9 or a nucleotide sequence represented by the 1124th to 1525th positions of the nucleotide sequence represented by SEQ ID NO:23 wherein from 1 to 10 bases of the nucleotide sequence are deleted, substituted and/or inserted, and which has promoter activity of the polynucleotide described in claim 4, (2) an expression vector comprising the polynucleotide described in (1), or (3) a cell transfected with the aforementioned expression vector.

**13.** Use of (1) a polynucleotide which comprises the polynucleotide described in claim 9 or a nucleotide sequence represented by the 1124th to 1525th positions of the nucleotide sequence represented by SEQ ID NO:23 wherein from 1 to 10 bases of the nucleotide sequence are deleted, substituted and/or inserted, and which has promoter activity of the polynucleotide described in claim 4, (2) an expression vector comprising the polynucleotide described in (1), or (3) a cell transfected with the aforementioned expression vector,
for screening a fibrosis improving agent.

**14.** A method for screening a fibrosis improving agent, **characterized in that** it comprises

(1) a step for allowing a cell transfected with an expression vector containing a polynucleotide which comprises (i) the polynucleotide described in claim 9 or (ii) a nucleotide sequence represented by 1124th to 1525th positions of the nucleotide sequence represented by SEQ ID NO:23 wherein from 1 to 10 bases of the nucleotide sequence are deleted, substituted and/or inserted, and which has promoter activity of the polynucleotide described in claim 4, to contact with a substance to be tested,
(2)a step for detecting the promoter activity, and
(3) a step for selecting a substance which accelerates the promoter activity.

**15.** A method for producing a medical composition for improving fibrotic conditions, **characterized in that** it comprises a step for carrying out screening using the screening method described in claim 14, and a step for preparing pharmaceutical preparations.

Fig. 1

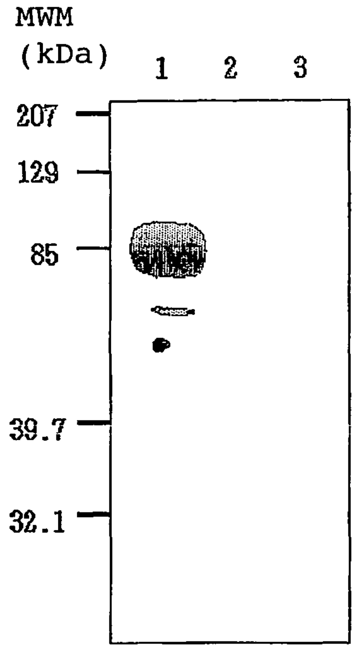


Fig. 2

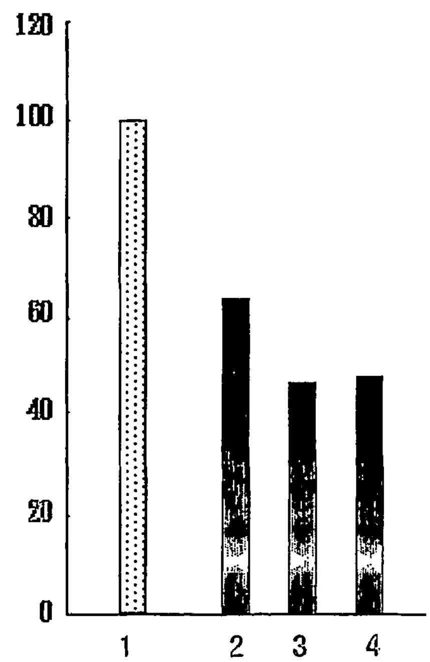

Fig. 3

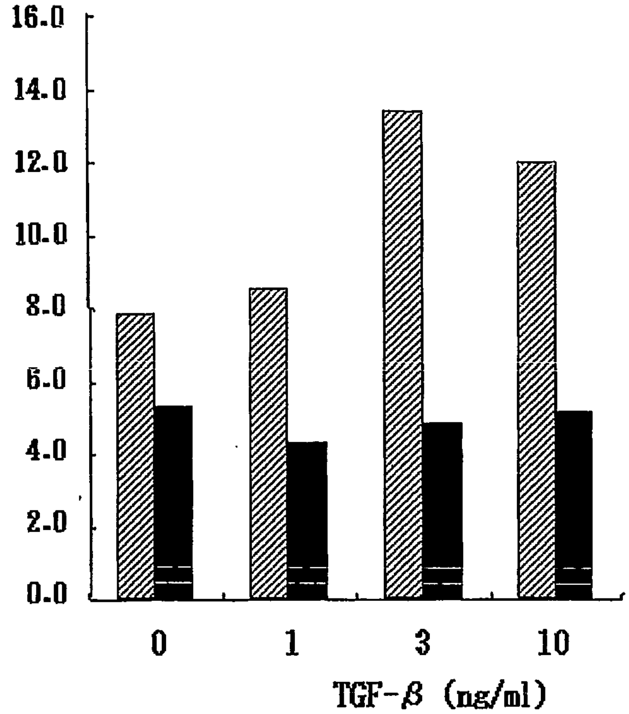
16.0
14.0
12.0
10.0
8.0
6.0
4.0
2.0
0.0
0
1
3
10
TGF-β (ng/ml)

Fig. 4

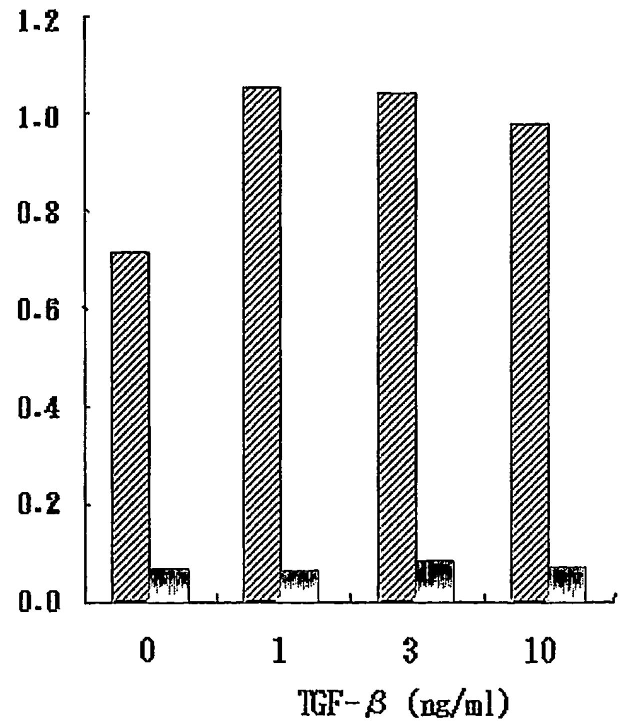
1.2
1.0
0.8
0.6
0.4
0.2
0.0
0
1
3
10
TGF-β (ng/ml)

Fig. 5

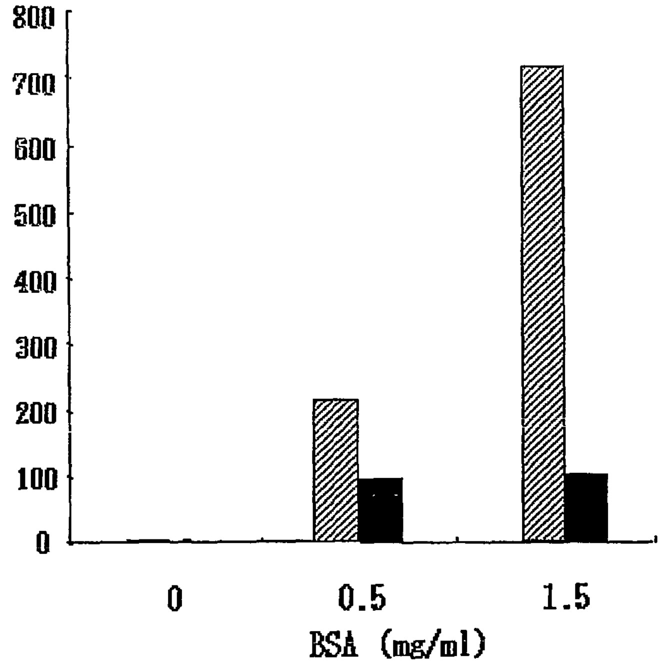
800
700
600
500
400
300
200
100
0
0
0.5
1.5
BSA (mg/ml)

Fig. 6

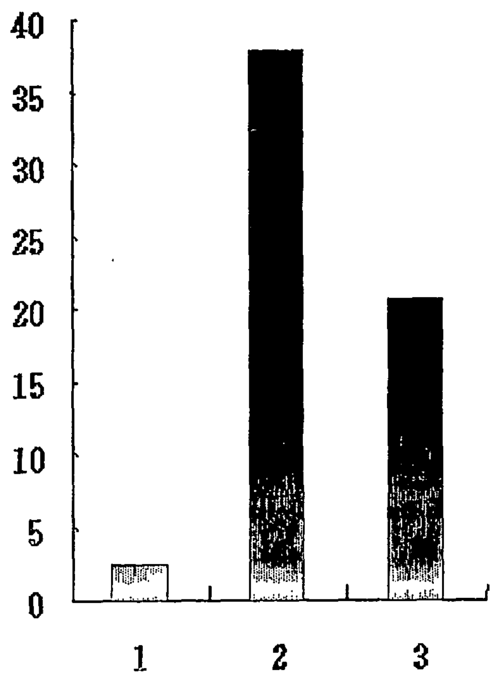
40
35
30
25
20
15
10
5
0
1
2
3

Fig. 7

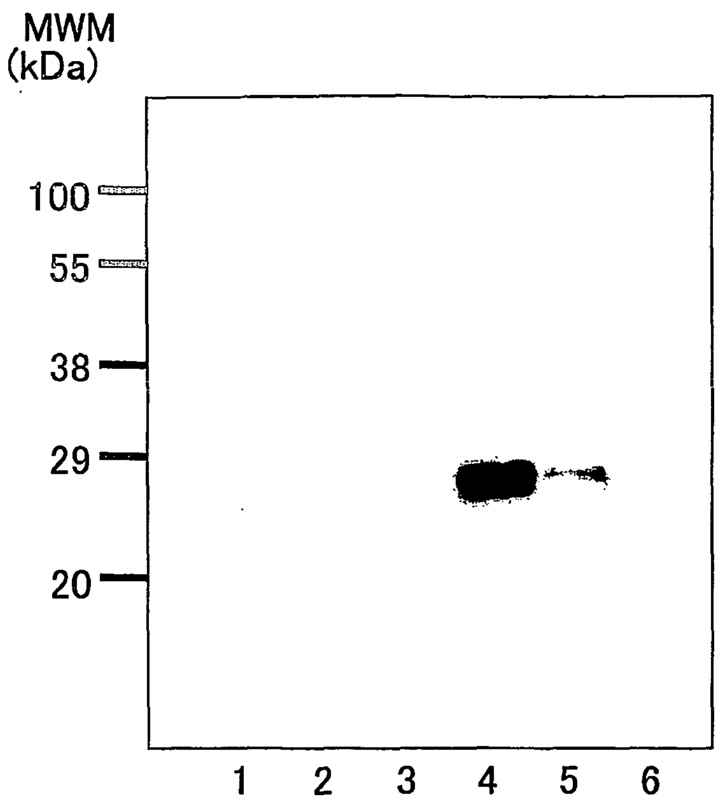

## INTERNATIONAL SEARCH REPORT

International application No.
PCT/JP2004/002648

A. CLASSIFICATION OF SUBJECT MATTER
Int.Cl$^7$ C07K14/47, C12N15/09, 1/15, 1/19, 1/21, 5/06, C12Q1/02, 1/68, A61K38/17, A61P13/00, 13/12, 43/00, G01N33/50, 33/15

According to International Patent Classification (IPC) or to both national classification and IPC

B. FIELDS SEARCHED
Minimum documentation searched (classification system followed by classification symbols)
Int.Cl$^7$ C07K14/00-16/46, C12N15/00-90, 1/00-7/08, C12Q1/00-70, A61K38/17, A61P13/00-12, 43/00, G01N33/50-98, 33/15

Documentation searched other than minimum documentation to the extent that such documents are included in the fields searched

Electronic data base consulted during the international search (name of data base and, where practicable, search terms used)
JICST FILE(JOIS), EUROPAT(QUESTEL), MEDLINE/BIOSIS/WPIDS(STN), SwissProt/PIR/GeneSeq, Genbank/EMBL/DDBJ/GeneSeq

C. DOCUMENTS CONSIDERED TO BE RELEVANT

| Category* | Citation of document, with indication, where appropriate, of the relevant passages | Relevant to claim No. |
|---|---|---|
| A | WO 2001/055315 A2 (HUMAN GENOME SCIENCE, INC.), 02 August, 2001 (02.08.01), & WO 2001/054474 A2 & US 2002/0090672 A1 & JP 2003-523760 A | 1-15 |
| P,A | WO 2003/025130 A2 (INCYTE GENOMICS, INC.), 27 March, 2003 (27.03.03), (Family: none) | 1-15 |
| A | M. H. BRANTON, et al., TGF-β and fibrosis, Microbes and Infection, 1999, 1, p. 1349-65 | 1-15 |
| A | D. A. CLARK, et al., Transforming growth factor-beta(TGF-β), The International Journal of Bio chemistry & Cell Biology, 1998, 30, p.293-8 | 1-15 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 April, 2004 (16.04.04) | 11 May, 2004 (11.05.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/002648

C (Continuation). DOCUMENTS CONSIDERED TO BE RELEVANT

| Category* | Citation of document, with indication, where appropriate, of the relevant passages | Relevant to claim No. |
|---|---|---|
| A | R. G. WELLS, Fibrogenesis V. TGF-β signaling pathways, Am. J. Physiol Gastrointest Liver Physiol, 2000, 279, G845-50 | 1-15 |
| A | H. YOKOI et al., Role of connective tissue growth factor in fibronectin expression and tubulointer stitial fibrosis, Am. J. Physiol Renal Physiol, 2002, 282, F933-42 | 1-15 |
| A | G. H. TESCH, et al., Monocyte chemoattractant protein-1 promotes mecrophage-mediated tubular injury, but not glomerular injury, in nephrotoxic serum nephritis, The Journal of Clinical Investigation, 1999, 103(1), pages 73 to 80 | 1-15 |
| A | J. J. GREELY, et al., Effects of Transforming Growth Factor-β on Collagen Synthesis by Normal Rat Kidney Epithelial Cells., American Journal of Pathology, 1992, 140(1), pages 45 to 55 | 1-15 |
| T | R. GOVINDEN, et al., Genealogy expression, and cellular function of transforming growth factor-β Pharmacology & Therapeutics, May, 2003, 98(2), p. 257-65 | 1-15 |

Form PCT/ISA/210 (continuation of second sheet) (January 2004)

**INTERNATIONAL SEARCH REPORT**

International application No.
PCT/JP2004/002648

**Box No. I Nucleotide and/or amino acid sequence(s) (Continuation of item1.b of the first sheet)**

1. With regard to any nucleotide and/or amino acid sequence disclosed in the international application and necessary to the claimed invention, the international search was carried out on the basis of:

   a. type of material
      - [x] a sequence listing
      - [ ] table(s) related to the sequence listing

   b. format of material
      - [ ] in written format
      - [x] in computer readable form

   c. time of filing/furnishing
      - [ ] contained in the international application as filed
      - [x] filed together with the international application in computer readable form
      - [ ] furnished subsequently to this Authority for the purposes of search

2. [x] In addition, in the case that more than one version or copy of a sequence listing and/or table relating thereto has been filed or furnished, the required statements that the information in the subsequent or additional copies is identical to that in the application as filed or does not go beyond the application as filed, as appropriate, were furnished.

3. Additional comments:

Form PCT/ISA/210 (continuation of first sheet (1)) (January 2004)

**INTERNATIONAL SEARCH REPORT**

International application No.
PCT/JP2004/002648

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: Parts of 3-8 and 12-15
because they relate to parts of the international application that do not comply with the prescribed requirements to such an extent that no meaningful international search can be carried out, specifically:

(See extra sheet.)

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest.
☐ No protest accompanied the payment of additional search fees.

Form PCT/ISA/210 (continuation of first sheet (2)) (January 2004)

**INTERNATIONAL SEARCH REPORT**

International application No.
PCT/JP2004/002648

Continuation of Box No.II-2 of continuation of first sheet(2)

Claims 1 to 8 and 12 to 15

Although EXAMPLES are examined, it is not proved that a polypeptide containing or consisting of the amino acid sequence represented by SEQ ID NO:4 suppresses the expression of type I collagen, fibronectin or MCP-1. Thus, the inventions according to claims 1 to 8 and 12 to 15 relating to this polypeptide are neither fully supported by the description nor clearly and completely disclosed therein to such an extent as allowing the inventions to be carried out by a person skilled in the art.

Claims 3 to 8 and 12 to 15

Although EXAMPLES are examined, it is not proved that "a polypeptide consisting of the amino acid sequence represented by the 1- to 166-positions in the amino acid sequence represented by SEQ ID NO:2" suppresses the expression of MCP-1. There is no common technical knowledge indicating that the amino acid sequence represented by SEQ ID NO:2 contains a part having a function of suppressing MCP-1 expression in its N-terminal. Thus, the inventions according to claims 3 to 8 and 12 to 15 relating to "a polypeptide consisting of the amino acid sequence represented by the 1- to 166-positions in the amino acid sequence represented by SEQ ID NO:2" are neither fully supported by the description nor clearly and completely disclosed therein to such an extent as allowing the inventions to be carried out by a person skilled in the art.

No search was made on the inventions relating to the following polypeptides which are neither fully disclosed in the description nor clearly and completely disclosed therein.

(1) A polypeptide consisting of an amino acid sequence derived from an amino acid sequence represented by the 1- to 166-positions in the amino acid sequence represented by SEQ ID NO:2 by deletion, substitution and/or insertion of 1 to 10 amino acids and suppressing the expression of MCP-1.

(2) A polypeptide containing the amino acid sequence represented by the 1- to 166-positions in the amino acid sequence represented by SEQ ID NO:2 and suppressing the expression of MCP-1.